# EUROPEAN PATENT SPECIFICATION

(11) **EP 3 662 922 B1**
(45) Date of publication and mention of the grant of the patent: **05.03.2025**
(21) Application number: 18840639.1
(22) Date of filing: 27.07.2018
(51) Int. Cl.: A61K 38/17, A61P 35/00, A61K 31/704, A61K 31/7048

(54) **A COMBINATION OF ENDOSTATIN AND A DNA DOUBLE STRAND BREAK INDUCING CHEMOTHERAPEUTIC AGENT FOR TREATING P53(-) MELANOMA AND P53(-) NON-SMALL CELL LUNG CANCER**
EINE KOMBINATION AUS ENDOSTATIN UND EINEM DNA-DOPPELSTRANGBRUCH-INDUZIERENDEN CHEMOTHERAPEUTISCHEN MITTEL ZUR BEHANDLUNG VON P53(-)-MELANOM UND P53(-)-NICHT-KLEINZELLIGEM LUNGENKREBS
COMBINAISON D'ENDOSTATINE ET D'UN AGENT CHIMIOTHÉRAPEUTIQUE INDUISANT UNE RUPTURE DE DOUBLE BRIN D'ADN POUR LE TRAITEMENT DU MÉLANOME P53(-) ET DU CANCER DU POUMON NON À PETITES CELLULES P53(-)

(30) Priority: 30.07.2017 CN 201710638033; 24.08.2017 CN 201710733900
(43) Date of publication of application: 10.06.2020
(73) Proprietor: Tsinghua University, Haidian District Beijing 100084 (CN); Beijing Protgen Ltd., Beijing 100085 (CN)
(72) Inventor: LUO, Yongzhang, Beijing 100084 (CN); JIA, Lin, Beijing 100084 (CN); CHANG, Guodong, Beijing 100085 (CN)
(74) Representative: Ström & Gulliksson AB
(86) International application number: PCT/CN2018/097609
(87) International publication number: WO 2019/024814

(56) References cited:
- EP-A1- 2 702 997
- TOULANY MAHMOUD ET AL: "Akt Promotes Post-Irradiation Survival of Human Tumor Cells through Initiation, Progression, and Termination of DNA-PKcs-Dependent DNA Double-Strand Break Repair", MOLECULAR CANCER RESEARCH, vol. 10, no. 7, 17 May 2012 (2012-05-17), US, pages 945 - 957, XP093039093, ISSN: 1541-7786, Retrieved from the Internet <URL:https://watermark.silverchair.com/945.pdf?token=AQECAHi208BE49Ooan9kkhW_Ercy7Dm3ZL_9Cf3qfKAc485ysgAAArkwggK1BgkqhkiG9w0BBwagggKmMIICogIBADCCApsGCSqGSIb3DQEHATAeBglghkgBZQMEAS4wEQQMS8NBDLns3ENa8mkXAgEQgIICbLthy8oG9flCgZpY00RtrQGudsE7Y5jMvZCbSA-j5LVb28kbGmbHfR6LDcL0xuoEKKyV88vXvmrKzIL9XHP-4ib3pe1nSGw1> DOI: 10.1158/1541-7786.MCR-11-0592
- ITASAKA ET AL: "Endostatin improves radioresponse and blocks tumor revascularization after radiation therapy for A431 xenografts in mice", INTERNATIONAL JOURNAL OF RADIATION: ONCOLOGY BIOLOGY PHYSICS, PERGAMON PRESS, USA, vol. 67, no. 3, 9 February 2007 (2007-02-09), pages 870 - 878, XP005897008, ISSN: 0360-3016, DOI: 10.1016/J.IJROBP.2006.10.030
- FANG PENG ET AL: "Recombinant Human Endostatin Normalizes Tumor Vasculature and Enhances Radiation Response in Xenografted Human Nasopharyngeal Carcinoma Models", PLOS ONE, vol. 7, no. 4, 9 April 2012 (2012-04-09), pages e34646, XP055039142, DOI: 10.1371/journal.pone.0034646
- VAN OORSCHOT BREGJE ET AL: "Targeting DNA double strand break repair with hyperthermia and DNA-PKcs inhibition to enhance the effect of radiation treatment", ONCOTARGET, vol. 7, no. 40, 1 September 2016 (2016-09-01), pages 65504 - 65513, XP093082321, DOI: 10.18632/oncotarget.11798
- "Applications of viruses for cancer therapy", vol. 115, 28 May 2013, ELSEVIER, ISBN: 978-0-12-398342-8, ISSN: 0065-230X, article KALIBEROV SERGEY A. ET AL: "Cancer Treatment with Gene Therapy and Radiation Therapy", pages: 221 - 263, XP093082328, DOI: 10.1016/B978-0-12-398342-8.00007-0
- WANG JINWAN ET AL: "[Results of randomized, multicenter, double-blind phase III trial of rh-endostatin (YH-16) in treatment of advanced non-small cell lung cancer patients] - PubMed", ZHONGGUO FEI AI ZA ZHI., 2005 AUG 20;8(4):283-90., 1 August 2005 (2005-08-01), XP055779140, Retrieved from the Internet <URL:https://pubmed.ncbi.nlm.nih.gov/21108883/> [retrieved on 20210223], DOI: 10.3779/j.issn.1009-3419.2005.04.07.
- YOU ZHEN Y ET AL: "Open Access PRIMARY RESEARCH The radiosensitization effects of Endostar on human lung squamous cancer cells H-520", CANCER CELL INTERNATIONAL, 24 May 2010 (2010-05-24), pages 17, XP055780018, Retrieved from the Internet <URL:https://cancerci.biomedcentral.com/track/pdf/10.1186/1475-2867-10-17.pdf> [retrieved on 20210226]
- SEARS CATHERINE R. ET AL: "Complex Cisplatin-Double Strand Break (DSB) Lesions Directly Impair Cellular Non-Homologous End-Joining (NHEJ) Independent of Downstream Damage Response (DDR) Pathways*", JOURNAL OF BIOLOGICAL CHEMISTRY, vol. 287, no. 29, 1 July 2012 (2012-07-01), pages 24263 - 24272, XP055779569, ISSN: 0021-9258, Retrieved from the Internet <URL:https://www.ncbi.nlm.nih.gov/pmc/articles/PMC3397852/pdf/zbc24263.pdf> DOI: 10.1074/jbc.M112.344911
- ANONYMOUS: "NCI-H520 [H520] ATCC HTB-182(TM) Homo sapiens lung squamous c", 1 January 2016 (2016-01-01), XP055780023, Retrieved from the Internet <URL:https://www.lgcstandards-atcc.org/Products/All/HTB-182.aspx?geo_country=nl#characteristics> [retrieved on 20210226]
- YANG FAN ET AL: "Anthracyclines induce double-strand DNA breaks at active gene promoters", MUTATION RESEARCH, vol. 773, 1 March 2015 (2015-03-01), AMSTERDAM, NL, pages 9 - 15, XP055779924, ISSN: 0027-5107, Retrieved from the Internet <URL:https://www.ncbi.nlm.nih.gov/pmc/articles/PMC4332850/pdf/nihms-658493.pdf> DOI: 10.1016/j.mrfmmm.2015.01.007
- MUNDT ARNO J ET AL: "Biologic Basis of Radiation Therapy - Holland-Frei Cancer Medicine - NCBI Bookshelf", HOLLAND-FREI CANCER MEDICINE. 6TH EDITION, 1 January 2003 (2003-01-01), XP055779144, Retrieved from the Internet <URL:https://www.ncbi.nlm.nih.gov/books/NBK12902/> [retrieved on 20210223]
- LAN TIAN ET AL: "Targeting hyperactivated DNA-PKcs by KU0060648 inhibits glioma progression and enhances temozolomide therapy via suppression of AKT signaling", ONCOTARGET, vol. 7, no. 34, 23 August 2016 (2016-08-23), pages 55555 - 55571, XP055779574, Retrieved from the Internet <URL:https://www.ncbi.nlm.nih.gov/pmc/articles/PMC5342436/pdf/oncotarget-07-55555.pdf> DOI: 10.18632/oncotarget.10864
- JIA, LIN ET AL.: "Endostatin Sensitizes p53-Deficient Non-Small- Cell Lung Cancer to Genotoxic Chemotherapy by Targeting DNA-Dependent Protein Kinase Catalytic Subunit", JOURNAL OF PATHOLOGY, vol. 243, no. 2, 31 October 2017 (2017-10-31), pages 255 - 266, XP055572118
- WANG, ZEXIN ET AL.: "Study on Endostatin Combined with Doxorubicin in the Treatment of H22 Hepatoma Transplanted Tumors in Mice", THE JOURNAL OF PRACTICAL MEDICINE, vol. 26, no. 6, 25 March 2010 (2010-03-25), pages 954 - 956, XP009519317, ISSN: 1006-5725
- CHEN, JIANHUA ET AL.: "Clinical Observation of Recombinant Human Endostatin Combined with Carboplatin and Etoposide for Advanced Small- Cell Lung Cancer", THE JOURNAL OF PRACTICAL MEDICINE, vol. 17, no. 5, 31 December 2013 (2013-12-31), pages 26 - 28, XP055675502
- LIU, FENGJUN ET AL.: "Gene Transfer of Endostatin Enhances the Efficacy of Doxorubicin to Suppress Human Hepatocellular Carcinomas in Mice", CANCER SCI., vol. 98, no. 9, 9 July 2007 (2007-07-09), pages 1381 - 1387, XP055572120
- YANG, MIN ET AL.: "Effect of Endostatin Combined with Adriamycin on the Proliferation and Apoptosis of Human Glioma Sarcomatosum Cell Line GL15 Cells", JOURNAL OF CLINICAL SURGERY, vol. 16, no. 6, 30 June 2008 (2008-06-30), XP055675504
- LU , SHUN ET AL.: "A Multicenter, Open-Label, Randomized Phase II Controlled Study of rh- Endostatin (Endostar) in Combination with Chemotherapy in Previously Untreated Extensive-Stage Small-Cell Lung Cancer", JOURNAL OF THORACIC ONCOLOGY, vol. 10, no. 1, 31 January 2015 (2015-01-31), pages 206 - 211, XP055572124
- XU, WEN ET AL.: "Endostar, a Recently Introduced Recombinant Human Endostatin, Inhibits Proliferation and Migration through Regulating Growth Factors, Adhesion Factors and Inflammatory Mediators in Choroid Retinal Endothelial Cells", MOLECULAR BIOLOGY, vol. 44, no. 4, 31 December 2010 (2010-12-31), pages 585 - 590, XP055572126

## Description

### Technical Field

The present disclosure relates to medicaments for treating tumor or cancer deficient in P53 function, in particular, a combination of endostatin and a chemotherapeutic agent for treating melanoma.

### Background

Tumor is a disease in which multiple factors play a role and are involved, and which develops in multiple stages. Chemotherapy plays an important role in the treatment of tumor. However, tumor cells are prone to develop resistance to chemotherapeutic agents, which seriously affects the treatment of cancer patients. Commonly used chemotherapeutic agents, such as alkylating agents, platinum complexes and topoisomerase inhibitors, use DNA as target and kill tumor cells by causing DNA damage to induce apoptosis. Therefore, DNA damage repair is considered to be an important reason for the resistance of tumor cells to chemotherapeutic agents. There are many forms of DNA damage caused by chemotherapeutic agents, including base damage, single-strand break (SSB), double-strand break (DSB), etc., among which DSB injury is the most serious. In mammalian cells, there are two main forms of DSB damage repair: Homologous Recombination (HR) and Nonhomologous End Joining (NHEJ). The HR repair process uses sister chromatid as template to accurately repair damaged DNA double strand, but only in the S and G2 phases of the cell cycle. Compared to HR, NHEJ is a more conservative repair mechanism that can be performed at any stage of the cell cycle. This repair does not require a homologous template, and uses protein interactions to directly connect damaged ends, which is the main mechanism for DSB repair in mammalian cells. The basic process of NHEJ is that after double-strand break occurs, Ku70/Ku80 binds to the end of the DSB injury site, and then Ku recruits DNA-PKcs to form a DNA-PK complex, which initiates the NHEJ repair mechanism. DNA-PKcs transmits repair signals through autophosphorylation and phosphorylation of downstream proteins. DNA terminal processing proteins (nucleases, polymerases, etc.) process the broken ends into structures suitable for ligation, and finally the ligase IV-XRCC4-XLF complex performs the ligation function. Among them, DNA-PKcs is the catalytic subunit of the DNA-PK complex, is a member of the phosphatidylinositol-3-kinase-like kinase family (PIKK) and plays a central role in NHEJ repair. Studies have shown that inhibition of DNA-PKcs can greatly enhance the sensitivity of tumor cells to DNA damaging agents (Elaine Willmore, et al. Blood. 2004; 103: 4659-4665, Yan Zhao, et al. Cancer Res 2006; 66(10): 5354-62). DNA-PK inhibitors are also under continuous development, and some DNA-PK inhibitors have entered clinical trials. It is a promising area to use a DNA repair inhibitor in combination with a chemotherapeutic agent to treat tumors.

P53 protein is considered to be one of the most famous tumor suppressors so far. It plays an important regulatory role in tumorigenesis and development, and is involved in multiple life activity processes such as cell cycle arrest, cell aging, DNA damage repair and apoptosis. Studies have shown that, under the stimulation of chemotherapeutic agents, wild-type P53 can activate caspase family proteins and cause tumor cell apoptosis by regulating a series of downstream target genes (Liz J Valente, et al. BioDiscovery 2013; 8: 3); however, the incidence of p53 gene abnormalities is high in tumors, including gene deletion or mutation. Abnormal p53 gene often causes loss of normal function, which in turn reduces the sensitivity of tumor cells to chemotherapeutic agents. Studies have shown that tumors with normal P53 function have a better response to chemotherapeutics agents than tumors with loss of P53 function (Toshiyuki Harada, et al. Cancer Sci 2003; 94: 394-399).

Endostatin (ES) is an endogenous neovascularization inhibitor discovered by a research group led by Professor Folkman from Harvard University in 1997. It was identified as the C-terminal fragment of collagen XVIII. It has shown activities *in vitro* and *in vivo* tests, such as inhibiting the proliferation of activated vascular endothelial cells, inhibiting the formation of new blood vessels, and inhibiting the development and metastasis of tumors, without cytotoxicity and developing drug resistance (O'Reilly MS et al. Cell 1997; 88:277-285; Boehm T. et al. Nature 1997; 390: 404-407). Subsequent studies have found that Endostatin also has a variety of biological activities, including inhibition of lymphatic endothelial cell proliferation, migration and lymphangiogenesis, inhibition of adipocyte differentiation and insulin resistance, etc. (Zhuo W et al. J Pathol 2010; 222, 249-260, Wang H et al. Diabetes 2015; 64, 2442-2456). But so far, there have been no reports showing that Endostatin regulates DNA damage repair pathways.

Wang Jinwan et al. (Results of randomized, multicenter, double-blind phase III trial of rh-endostatin (YH-16) in treatment of advanced non-small cell lung cancer patients, Zhongguo Fei Ai Za Zhi, 2005, 8, 4: 233-290) discloses a combination therapy of endostatin and cisplatin for non-small cell lung cancer.

You Zhen Y et al. (Open access PRIMARY RESEARCH The radiosensitization effects of Endostar on human lung squamous cancer cells H-520, Cancer Cell International, 2010, page 17) discloses that recombinant human endostatin increases the radiosensitivity and apoptosis of human lung squamous cancer cell line H-520 in vitro.

Sears Catherine R. et al. (Complex cisplatin-double strand break (DSB) lesions directly impair cellular non-homologous end-joining (NHEJ) independent of downstream damage response (DDR) pathways, Journal of Biological Chemistry, 2012, 287, 29: 24263-24272) discloses cisplatin as a DNA double strand breaker.

"NCl-H520 [H520] ATCC HTB-182(TM) Homo sapiens lung squamous c" (2016) (retrieved from the internet: URL:https://www.lgcstandards-atcc.org/Products/AII/HTB-182.aspx?geo country=nl#characteristics) discloses that H-520 cell line has reduced p53 m RNA levels relatives to the normal lung tissue.

Yang Fan et al. (Anthracyclines induce double-strand DNA breaks at active gene promoters, Mutation Research, 2015, 773: 9-15) discloses Doxorubicin and Etoposide as DNA-DSBs

EP2702997A1 discloses an endostatin derivative having (M)GGSHHHHH at its N-terminus and an endostatin derivative modified with mPEG-ALD at the N-terminal α-amine.

### Summary

The present invention is defined in the appended claims.

The present disclosure provides a medicament for treating tumor or cancer deficient in P53 function by using an endostatin in combination with a chemotherapeutic agent for inducing DNA double-strand break. The composition of the present disclosure can be used to treat tumor or cancer with loss of P53 function.

According to another aspect of the present disclosure, a combination of drugs is provided, including an endostatin and a chemotherapeutic agent, wherein the chemotherapeutic agent is a chemotherapeutic agent that induces DNA double-strand break, wherein the combination of drugs is used for treating tumor or cancer deficient in P53 function, wherein the tumor or cancer is melanoma.

In some embodiments, the chemotherapeutic agent is Etoposide or Doxorubicin.

In some embodiments, the tumor or cancer is melanoma.

In some embodiments, the endostatin and the chemotherapeutic agent are administered simultaneously or sequentially.

In some embodiments, the endostatin is:
a natural human endostatin;
an endostatin variant obtained by adding 9 additional amino acids MGGSHHHHH (SEQ ID NO: 1) to the N-terminus of the natural human endostatin, wherein the Met at the N-terminus of the endothelin variant is sometimes partially deleted when expressed by E. coli; or
a product obtained by modifying a natural human endostatin with a monomethoxy polyethylene glycol propionaldehyde (mPEG-ALD) with a molecular weight of 20 kDa, wherein their coupling site is the activated mPEG-ALD aldehyde group and the N-terminal α-amino group of the natural human endostatin.

According to another aspect of the present disclosure, endostatin for use in increasing sensitivity of a tumor or cancer cell to a chemotherapeutic agent for inducing DNA double-strand break is provided, wherein the cell is deficient in P53 function, wherein the tumor or cancer is melanoma.

In some embodiments, the step of contacting the cell with an endostatin is performed simultaneously or sequentially with the treatment regimen for inducing DNA double-strand break.

In some embodiments, the cell is contacted with an endostatin prior to the treatment regimen for inducing DNA double-strand break.

In some embodiments, the cell is contacted with an endostatin after the treatment regimen for inducing DNA double-strand break.

In some embodiments, the chemotherapeutic agent is Etoposide or Doxorubicin.

In some embodiments, the endostatin is:
a natural human endostatin;
an endostatin variant obtained by adding 9 additional amino acids MGGSHHHHH (SEQ ID NO: 1) to the N-terminus of the natural human endostatin, wherein the Met at the N-terminus of the endothelin variant is sometimes partially deleted when expressed by E. coli; or
a product obtained by modifying a natural human endostatin with a monomethoxy polyethylene glycol propionaldehyde (mPEG-ALD) with a molecular weight of 20 kDa, wherein their coupling site is the activated mPEG-ALD aldehyde group and the N-terminal α-amino group of the natural human endostatin.

According to another aspect of the present disclosure, a combination comprising a chemotherapeutic agent and an endostatin for use in inducing apoptosis in a tumor or cancer cell deficient in P53 function is provided, wherein the tumor or cancer is melanoma..

In some embodiments, the step of inducing DNA double-strand break in a cell is performed simultaneously or sequentially with the step of contacting the cell with an endostatin.

In some embodiments, the cell is contacted with an endostatin prior to inducing DNA double-strand break in the cell.

In some embodiments, the cell is contacted with an endostatin after inducing DNA double-strand break in the cell.

In some embodiments, the chemotherapeutic agent is Etoposide or Doxorubicin.

In some embodiments, the endostatin inhibits the repair of DNA double strand break induced by the chemotherapeutic agent in the tumor or cancer cell.

In some embodiments, the endostatin is:
a natural human endostatin;
an endostatin variant obtained by adding 9 additional amino acids MGGSHHHHH (SEQ ID NO: 1) to the N-terminus of the natural human endostatin, wherein the Met at the N-terminus of the endothelin variant is sometimes partially deleted when expressed by E. coli; or
a product obtained by modifying a natural human endostatin with a monomethoxy polyethylene glycol propionaldehyde (mPEG-ALD) with a molecular weight of 20 kDa, wherein their coupling site is the activated mPEG-ALD aldehyde group and the N-terminal α-amino group of the natural human endostatin.

### Brief Description of The Drawings

Figure 1A: Coomassie blue-staining showed the Endostatin-binding proteins in tumor cells as detected by SDS-PAGE. CNBr represents hydrogen bromide-activated agarose gel 4B affinity medium.
Figure 1B: The Endostatin-binding proteins in tumor cells as detected by immunoblotting assay.
Figure 1C: The interaction of Endostatin with DNA-PKcs in tumor cells as detected by co-immunoprecipitation assay.
Figure 2A: Immunoblotting assay showed that Endostatin inhibited the activation of DNA-PKcs signaling pathway in H1299 cells induced by the chemotherapeutic agent.
Figure 2B: Immunoblotting assay showed that Endostatin inhibited the activation of DNA-PKcs signaling pathway in A549 cells induced by the chemotherapeutic agent.
Figure 3A: Immunoblotting assay showed that Endostatin delayed DNA-PKcs-mediated DNA damage repair in H1299 cells.
Figure 3B: Immunoblotting assay showed that Endostatin delayed DNA-PKcs-mediated repair of DNA damage in A549 cells.
Figure 4A: Endostatin synergized with Etoposide promoted the apoptosis of H1299 cells, which depended on the presence of DNA-PKcs. ** represents P < 0.01; ns, no significant difference.
Figure 4B: Endostatin synergized with Etoposide promoted the apoptosis of A549 cells, which depended on the presence of DNA-PKcs. ** represents P < 0.001; ns, no significant difference.
Figure 5A: Endostatin synergized with Etoposide inhibited the activity of H1299-NC cells. * represents P < 0.05; *** represents P < 0.001.
Figure 5B: Endostatin cannot enhance the inhibitory effect of Etoposide on the activity of H1299-P53 cells.
Figure 5C: Endostatin cannot enhance the inhibitory effect of Etoposide on the activity of A549-NC cells.
Figure 5D: Endostatin synergized with Etoposide inhibited the activity of A549-shP53 cells. * represents P < 0.05; ** represents P < 0.01.
Figure 6A: Endostatin synergized with Etoposide inhibited the survival of H1299-NC cells. *** represents P < 0.001.
Figure 6B: Endostatin cannot enhance the inhibitory effect of Etoposide on the survival of H1299-P53 cells. ns, no significant difference.
Figure 6C: Endostatin cannot enhance the inhibitory effect of Etoposide on the survival of A549-NC cells. ns, no significant difference.
Figure 6D: Endostatin synergized with Etoposide inhibited the survival of A549-shP53 cells. *** represents P < 0.001.
Figure 7A: Endostatin synergized with Etoposide promoted apoptosis of H1299-NC cells. *** represents P < 0.001.
Figure 7B: Endostatin cannot enhance Etoposide to promote the apoptosis of H1299-P53 cells. ns, no significant difference.
Figure 7C: Endostatin cannot enhance Etoposide to promote the apoptosis of A549-NC cells. ns, no significant difference.
Figure 7D: Endostatin synergized with Etoposide inhibited the apoptosis of A549-shP53 cells. *** represents P < 0.001.
Figure 8A: Endostatin synergized Doxorubicin inhibited the activity of H1299-NC cells. * represents P < 0.05; ** represents P < 0.01.
Figure 8B: Endostatin cannot enhance the inhibitory effect of Doxorubicin on the activity of H1299-P53 cells.
Figure 8C: Endostatin cannot enhance the inhibitory effect of Doxorubicin on the activity of A549-NC cells.
Figure 8D: Endostatin synergized Doxorubicin inhibited the activity of A549-shP53 cells. * represents P < 0.05; *** represents P < 0.001.
Figure 9A: Endostatin synergized with Etoposide inhibited the activity of MDA-MB-43 5 S cells. * represents P < 0.05; ** represents P < 0.01.
Figure 9B: Endostatin cannot enhance the inhibitory effect of Etoposide on the activity of A375 cells.
Figure 10A: Immunoblotting assay showed that Endostatin inhibited the repair of cell damage by DNA-PKcs, and promoted the activation of apoptotic pathway and degradation of DNA-PKcs in H1299-NC cells.
Figure 10B: Immunoblotting assay showed that Endostatin had no effect on DNA damage, activation of apoptotic pathway, and degradation of DNA-PKcs in H1299-P53 cells induced by Etoposide.
Figure 10C: Immunoblotting assay showed that Endostatin had no effect on DNA damage, activation of apoptotic pathway, and degradation of DNA-PKcs in A549-NC cells induced by Etoposide.
Figure 10D: Immunoblotting assay showed that Endostatin inhibited the repair of cell damage by DNA-PKcs, and promoted the activation of apoptotic pathway and degradation of DNA-PKcs in A549-shP53 cells.
Figure 11A: Endostatin synergized with Etoposide up-regulated mRNA expression levels of pro-apoptotic molecules *BAX, NOXA, PUMA, P21* in H1299-NC cells. * represents P <0.05; ** represents P <0.01; *** represents P <0.001.
Figure 11B: Endostatin had no effect on mRNA expression levels of pro-apoptotic molecules *BAX, NOXA, PUMA, P21* in H1299-P53 cells up-regulated by Etoposide. ns, no significant difference.
Figure 11C: Endostatin had no effect on the mRNA expression levels of pro-apoptotic molecules *BAX, NOXA, PUMA, P21* in A549-NC cells up-regulated by Etoposide. ns, no significant difference.
Figure 11D: Endostatin synergized with Etoposide up-regulated mRNA expression levels of pro-apoptotic molecules *BAX, NOXA, PUMA, P21* in A549-shP53 cells. * represents P <0.05; ** represents P <0.01.
Figure 12A: Endostatin enhanced the inhibitory effect of Etoposide on H1299-NC tumor growth. ** represents P < 0.01.
Figure 12B: Endostatin cannot enhance the inhibitory effect of Etoposide on H1299-P53 tumor growth. ns, no significant difference.
Figure 12C: Endostatin cannot enhance the inhibitory effect of Etoposide on A549-NC tumor growth. ns, no significant difference.
Figure 12D: Endostatin enhanced the inhibitory effect of Etoposide on A549-shP53 tumor growth. ** represents P < 0.01.
Figure 13A: The combined use of Endostatin and Etoposide had a synergistic effect on prolonging the survival time of H1299 tumor-bearing mice. * represents P < 0.05.
Figure 13B: The combined use of Endostatin and Etoposide cannot further prolong the survival time of A549 tumor-bearing mice in the group treated with Etoposide alone. ns, no significant difference.
Figure 14A: Immunofluorescence method showed that Endostatin synergized with Etoposide inhibited the proliferation of H1299-NC tumor and the expression level of DNA-PKcs. * represents P < 0.05; ** represents P < 0.01.
Figure 14B: Immunofluorescence method showed that Endostatin could not enhance the inhibitory effect of Etoposide on the proliferation of H1299-P53 tumor and the expression level of DNA-PKcs. ns, no significant difference.
Figure 14C: Immunofluorescence method showed that Endostatin could not enhance the inhibitory effect of Etoposide on the proliferation of A549-NC tumor and the expression level of DNA-PKcs. ns, no significant difference.
Figure 14D: Immunofluorescence method showed that Endostatin synergized with Etoposide inhibited the proliferation of A549-shP53 tumor and the expression level of DNA-PKcs. * represents P < 0.05; ** represents P < 0.01.
Figure 15A: Immunoblotting assay showed that ZBP-Endostatin and PEG-Endostatin inhibited the activation of DNA-PKcs signaling pathway in H1299 cells induced by the chemotherapeutic agent.
Figure 15B: Immunoblotting assay showed that ZBP-Endostatin and PEG-Endostatin inhibited the activation of DNA-PKcs signaling pathway in A549 cells induced by the chemotherapeutic agent.

### Detailed Description

It is found in the present disclosure that an endostatin can directly act on tumor cells and regulate the sensitivity of tumor cells to chemotherapeutic agents. It is found in *in vitro* cytological experiments that an endostatin can significantly enhance the inhibitory effect of chemotherapeutic agents on P53-deficient tumor cell activity and cell colony-forming ability, and promote tumor cell apoptosis induced by the chemotherapeutic agents. Meanwhile, in a mouse xenograft tumor model, an endostatin can significantly enhance the growth inhibiting effect of chemotherapeutic agents on P53-deficient non-small cell lung cancer xenografts and prolong the survival of tumor-bearing mice.

In the present disclosure, in order to explore the mechanism by whichan endostatin regulates the sensitivity of tumor cells to chemotherapy, by screening proteins that interact with an endostatin in tumor cells, it was found that the endostatin can specifically interact with DNA-PKcs. DNA-PKcs is a key protein in the non-homologous end joining pathway in mammalian cells and is involved in the repair of DNA double-strand breaks in cells. The interaction between an endostatin and DNA-PKcs can inhibit its activity and further inhibit DNA damage repair. P53-deficient tumor cells show resistance to chemotherapeutic agents, and cell survival depends on the DNA-PKcs-mediated DNA repair process. In this situation, endostatin causes the accumulation of DNA damage in cells by inhibiting the activity of DNA-PKcs, and then induces apoptosis and enhances the therapeutic effect of chemotherapeutic agents. Cells with normal P53 function are more sensitive to chemotherapeutic agents. P53 activates Caspase-3-mediated apoptosis under the treatment of a chemotherapeutic agent alone, and DNA-PKcs as a substrate of Caspase-3 will also be cleaved and reduced during this process. Therefore, the effect of using the endostatin to inhibit DNA-PKcs is very limited.

The action mechanism of an endostatin in P53-deficient tumor cells can be understood as follows. Since P53 can activate caspase family proteins, and cause tumor cell apoptosis by regulating a series of downstream target genes, P53-deficient tumor cells have a higher apoptosis threshold, and with the stimulation by DNA damage inducers such as Etoposide, the DNA-PKcs signaling pathway is activated and mediates DNA damage repair through non-homologous end-joining pathway. In this situation, endostatin can directly bind to DNA-PKcs and inhibit its activity, causing accumulation of DNA damage in cells, and the cells develop from DNA repair to apoptosis. At this time, the activation of Caspase pathway in turn leads to the degradation of DNA-PKcs, and further blocks DNA repair and promotes cell death. In contrast, during DNA damage, tumor cells with normal P53 function have lower apoptosis threshold, and P53 will trigger the activation of apoptosis-related effectors. In this situation, in the early stage of apoptosis, DNA-PKcs is degraded by Caspase-3 activated by the P53 pathway, which further weakens DNA repair. Therefore, the P53 signal pathway itself can form a feedback regulatory loop. The effect of increasing the chemotherapy sensitivity is not ideal by targeting endothelin to DNA-PKcs in tumor cells with normal P53 function.

Therefore, the present disclosure is essentially based on the discovery that an endostatin can directly bind to DNA-PKcs and inhibit its activity, that is, the endostatin is an inhibitor of DNA-PKcs. For P53-deficient cells, when DNA damage is induced by any treatment method (such as a chemotherapeutic agent or radiotherapy), endostatin can promote cell apoptosis through the above-mentioned mechanism, thereby increasing the sensitivity of cells to the treatment method. Even for cells with normal P53 function, the use of endostatin can reduce the dose of a chemotherapeutic agent or the dose of radiotherapy. At this time, although the DNA damage of cells is reduced due to the decrease in the dose of a chemotherapeutic agent or the dose of radiotherapy, the endostatin can inhibit the activity of DNA-PKcs, thereby promoting the accumulation of DNA damage in cells and promoting apoptosis. Therefore, the effect of an original dose can still be achieved when the dose of a chemotherapeutic agent or the dose of radiotherapy is reduced, thereby reducing the side effects of the chemotherapeutic agent or radiotherapy and improving its compliance.

"Endostatin (ES)" as described herein has its broadest meanings and includes a variety of proteins with natural endostatin activities, such as the activities of inhibiting the vascular endothelial cell proliferation, migration and angiogenesis *in vivo.*

The "endostatin" as described herein may be a natural endostatin, preferably a natural human endostatin, but is not limited thereto. For example, it may be a natural endostatin from other mammals, such as mouse, rat, pig, dog, rabbit, sheep, goat, cat, and the like. The endostatin may be purified from a natural source, or be a recombinant protein.

The sequence of the natural human endostatin used in the present disclosure may be, for example:
MHSHRDFQPVLHLVALNSPLSGGMRGIRGADFQCFQQARAVGLAGTFRAFLSSR LQDLYSIVRRADRAAVPIVNLKDELLFPSWEALFSGSEGPLKPGARIFSFDGKDVLRH PTWPQKSVWHGSDPNGRRLTESYCETWRTEAPSATGQASSLLGGRLLGQSAASCHH AYIVLCIENSFMTASK (SEQ ID NO: 2); wherein the Met at the N-terminus is sometimes partially deleted when it is expressed by *E. coli.*

The "endostatin" as described herein may also be a functional variant of natural endostatin, such as an engineered functional variant, which has substitution, deletion or addition of one or several amino acids compared with natural endostatin, and has basically the same biological functions, such as the activities of inhibiting the proliferation, migration and *in vivo* angiogenesis of vascular endothelial cells.

The term "functional variant" as used herein includes mutants of endostatin that contain substitutions, deletions or additions of one or more (for example, 1-5, 1-10, or 1-15, in particular, , such as 1, 2, 3, 4, 5, 6, 7, 8, 9, 10, 11, 12, or more) amino acids in the amino acid sequence, and the mutants have biological activities similar to those of endostatin to inhibit the proliferation, migration and *in vivo* angiogenesis of vascular endothelial cells. The biological activities of a "functional variant" of endostatin can be, for example, such as 30% or higher, 50% or higher, 60% or higher, 70% or higher, 80% or higher or 90% or higher of those of a natural endostatin, for example, a natural human endostatin. The "functional variant" may be a naturally occurring mutant or an artificial mutant, such as a mutant obtained by site-directed mutagenesis, or a mutant produced by a genetic recombination method.

The biological activities of the "functional variant" can be detected by methods well known in the art for detecting endostatin activities. For example, HMEC cells can be selected, and the inhibition rate of HMEC cell migration by a functional variant is analyzed using the Migration (Tranwell Assay) method, and the number of cells is counted to reflect the protein activity (see Luo yongzhang et al., Endostatin inhibits tumourlymphangiogenesis and lymphatic metastasis via cell surface nucleolin on lymphangiogenic endothelial cells (J Pathol 2010; 222: 249-260)).

In some embodiments of the present disclosure, the "endostatin" may be a functional variant of natural human endostatin, such as ZBP-Endostatin (under Trade Name of Endostar), which is an ES variant obtained by adding 9 additional amino acids (MGGSHHHHH) to the N-terminus of natural human ES, in order to increase soluble expression and facilitate purification. The amino acid sequence of ZBP-Endostatin is:
MGGSHHHHHHSHRDFQPVLHLVALNSPLSGGMRGIRGADFQCFQQARAVGLA GTFRAFLSSRLQDLYSIVRRADRAAVPIVNLKDELLFPSWEALFSGSEGPLKPGARIFS FDGKDVLRHPTWPQKSVWHGSDPNGRRLTESYCETWRTEAPSATGQASSLLGGRLL GQSAASCHHAYIVLCIENSFMTASK (SEQ ID NO: 3); wherein the Met at its N-terminus is sometimes partially deleted when it is expressed by E. coli.

Other examples of endostatin functional variants include those endostatin mutants disclosed in the PCT International Application PCT/CN2012/081210, such as ES006, ES008, ES011, S02, S09, Z006, Z008, ZN1, etc.

The "endostatin" as described herein may also be a derivative or a modified product (such as a polyethylene glycol modified product) of natural endostatin or a functional variant thereof, which has substantially the same biological functions as natural endostatin, such as the activities of inhibiting the proliferation, migration and *in vivo* angiogenesis of vascular endothelial cells.

In some embodiments of the present disclosure, the "endostatin" may be a derivative of natural human endostatin, for example, a product resulted from the modification of a natural human ES with a monomethoxypolyethylene glycol propionaldehyde (mPEG-ALD) with a molecular weight of 20 kDa, wherein the coupling site is the activated mPEG-ALD aldehyde group and the N-terminal α-amino.

The "DNA-dependent protein kinase catalytic subunit (DNA-PKcs)" as described herein is a catalytic subunit of the DNA-PK complex, is a member of the phosphatidylinositol-3-kinase-like kinase family (PIKK) and plays a central role in nonhomologous end joining (NHEJ) repair.

The "treatment regimen for inducing DNA double-strand break" as described herein can be any form of treatment regimen as long as the treatment regimen can finally result in DNA double-strand break. The treatment regimen may be, for example, a treatment regime that directly causes a double-strand break in DNA. The treatment regimen may also be, for example, a treatment regimen in which a single-strand break in DNA occurs, thereby finally leading to a double-strand break in DNA. The treatment regimen may also be, for example, a treatment regimen that causes chromosomal damage, thereby finally leading to DNA double-strand break. The treatment regimen may also be a treatment regimen that causes DNA double-strand break, for example by DNA-PKcs inhibition. The treatment regimen may be radiotherapy, or a chemotherapeutic agent, may be one chemotherapeutic agent or a combination of more different chemotherapeutic agents, or may be a combination treatment regimen of one or more chemotherapeutic agents and radiotherapy.

Radiotherapy regimens for inducing DNA double-strand break are well known in the art. Radiotherapy can be performed by any suitable technique, including but not limited to electromagnetic radiation or ionizing radiation. The electromagnetic radiation may be, for example, X-rays, gamma rays; and the ionizing radiation may be, for example, an electron beam or the like. Dosimetry for radiotherapy is also well known in the art.

The term "chemotherapeutic agent" as used herein refers to a compound that has a therapeutic effect on tumor or cancer. The chemotherapeutic agent that can be used in the present disclosure is a chemotherapeutic agent that can induce DNA double-strand break. For example, it may be a topoisomerase II inhibitor, an alkylating agent, a platinum complex, bleomycin, or doxorubicin, such as, but not limited to, Etoposide or Doxorubicin.

In the present disclosure, an endostatin can be used in combination with a treatment regimen for inducing DNA double-strand break, for treating tumor or cancer. The tumor or cancer is a tumor or cancer suitable for treatment with a treatment regime for inducing DNA double-strand break.

The "p53" as described herein is a tumor suppressor gene that encodes a protein with a molecular weight of 53 kDa, called P53. The biological function of normal P53 is similar to that of the "guardian of the genome", which checks for DNA damage site at G1 phase and monitors genome integrity. If there is damage, the P53 protein prevents DNA replication to provide enough time for the damaged DNA to be repaired. If the repair fails, the P53 protein will trigger apoptosis. If two copies of the p53 gene are mutated, which causes the P53 protein to lose its normal function, the proliferation of cells will be out of control and the cells will become cancerous.

The tumor or cancer that can be treated using the agent or treatment regimen described herein may be deficient in P53 function. The tumor or cancer with loss of P53 function include tumor or cancer with loss of P53 function in some or all of the cells. The cells with loss of P53 function may be cells with complete loss of P53 function or cells with partial loss of P53 function. The complete loss of P53 function may be, for example, complete loss of the biological function of P53 compared to a cell in which P53 is wild-type. The partial loss of P53 function may be, for example, partial loss or attenuation of the biological function of P53 compared to a cell in which P53 is wild-type. A cell with a loss of P53 function may have, for example, a decreased or disrupted expression and/or activity of P53 protein compared to a normal cell, which can be caused, for example, by a mutation in its coding nucleic acid, or by a mutation in the nucleic acid encoding its regulator. The decreased or disrupted expression and/or activity of P53 protein can be manifested as absence of P53 protein, complete inactivation of P53 protein, decrease in the amount of P53 protein, or decrease in the function of P53 protein. The "mutation" may include insertion, deletion or substitution of one or more nucleotides.

In cells with complete loss of P53 function, they are not sensitive to radiotherapy or chemotherapeutic agents. This is because although radiotherapy or chemotherapy agents cause DNA damage in cells, which activates the DNA-PKcs signaling pathway mediates DNA damage repair through non-homologous end joining pathways, due to the loss of P53 function, Caspase is not easily activated. In the presence of DNA-PKcs, damage can be repaired in time, which makes the cells resistant to the treatment regimen to some extent and does not lead to apoptosis easily. Endostatin can inhibit the activity of DNA-PKcs, promote the accumulation of DNA damage in cells, and promote apoptosis. Therefore, for cells with loss of function of P53, especially tumor or cancer cells, which are treated with a treatment regimen for inducing DNA double-strand break such as radiotherapy or a chemotherapy agent, endostatin can increase the sensitivity of these cells to the treatment regimen for inducing DNA double-strand break, and promote the therapeutic effects of the treatment regimen for inducing DNA double-strand break.

For cells without loss of P53 function (that is, P53 functions normally), the cells are more sensitive to radiotherapy or a chemotherapy agent. In the early stage of apoptosis, DNA-PKcs will be degraded by Caspase-3 activated by the P53 pathway, and endostatin does not significantly increase its sensitivity. However, since endostatin can inhibit DNA-PKcs activity, the use of endostatin can reduce the dose of a treatment regimen for inducing DNA double-strand break such as radiotherapy or a chemotherapeutic agent. The "reduce the dose of a treatment regimen for inducing DNA double-strand break" refers to that when compared with the dose of a treatment regimen for inducing DNA double-strand break used alone, the dose of the treatment regimen for inducing DNA double-strand break can be smaller when used in combination with endostatin, but can also achieve the same therapeutic effect. In other words, when the treatment regimen for inducing DNA double-strand break is used in combination with endostatin, its dose applied does not exceed the dose usually applied when the treatment regimen for inducing DNA double-strand break is used alone.

When the treatment regimen for inducing DNA double-strand break is radiotherapy, the dose refers to a radiation dose. The dose usually applied when the radiotherapy is performed alone may be, for example, 1-100 Gy. When the radiotherapy is used in combination with endostatin, the radiation dose can be reduced by 1%, 5%, 10%, 20%, 30%, 40%, 50%, 60%, 70%, 80%, or even 90% compared with the one when the radiotherapy is applied alone.

When the treatment regimen for inducing DNA double-strand break is a chemotherapeutic agent, the dose refers to an administration dose of the chemotherapeutic agent. When a chemotherapeutic agent is used in combination with endostatin, the dose of the chemotherapeutic agent can be reduced by 1%, 5%, 10%, 20%, 30%, 40%, 50%, 60%, 70%, 80%, or even 90% compared with the one when the chemotherapy agent is administrated alone.

For cells with partial loss of P53 function, which are treated with a treatment regimne for inducing DNA double-strand break, vascular endothelial inhibition can have both of the above effects, which can not only increase the sensitivity of these cells to the treatment regimen for inducing DNA double-strand break and promote the therapeutic effect of the treatment regimen for inducing DNA double-strand break, but also reduce the dose of the treatment regimen for inducing DNA double-strand break.

The amount of endostatin in the pharmaceutical composition, combination drugs or kit of the present disclosure, or the amount of endostatin in the use or method of the present disclosure, is an amount capable of effectively inhibiting DNA-PKcs in tumor cells.

The tumors described herein include benign and malignant tumors, and doubling of tumor cells refers to uncontrolled and progressive histological cell growth. Some of these growths are benign, but others are "malignant" and can cause the death of organism. In addition to exhibition of aggressive cell proliferation, malignant tumors can infiltrate surrounding tissues and metastasize. The cancers described herein generally include malignant solid tumors, as well as leukemia, lymphoma, and other cancers that do not usually exist as tumor masses but are distributed in the blood vessels or lymphoreticular system.

The treatment of tumors or cancers by using the regimen of the present disclosure is achieved by inducing apoptosis. Therefore, the therapeutic effect of the regimen of the present disclosure on tumor or cancer is not closely related to the origin of tumor cells or cancer cells, but is related to the molecular typing of cells. For example, tumors or cancers suitable for treatment by the method of the present disclosure may be P53-deficient. Tumors or cancers that can be treated with the regimen of the present disclosure include, but are not limited to, solid tumors/malignant tumors, myxoid and round cell carcinomas, locally advanced tumors, metastatic cancers, human soft tissue sarcomas (including Ewing's sarcoma), cancer metastases (including lymphatic metastases), squamous cell carcinoma (especially head and neck squamous cell carcinoma, esophageal squamous cell carcinoma), oral cancer, hematological malignancies (including multiple myeloma), leukemia (including acute lymphocytic leukemia, acute nonlymphocytic leukemia, chronic lymphocytic leukemia, chronic myeloid leukemia, and hairy cell leukemia), exudative lymphoma (body cavity-based lymphoma), thymic lymphoma, lung cancer (including small cell cancer), skin T-cell lymphoma, Hodgkin's lymphoma, non-Hodgkin's lymphoma, adrenocortical cancer, ACTH-producing tumor, non-small cell lung cancer, breast cancer (including small cell and ductal cancer), gastrointestinal cancer (including gastric cancer, colon cancer, colorectal cancer), polyps associated with colorectal tumor formation, pancreatic cancer, liver cancer, urinary cancer (including bladder cancer, including primary superficial bladder tumors, invasive transitional cell carcinoma of bladder and muscular invasive bladder cancer), prostate cancer, malignant tumors of female reproductive tract (including ovarian cancer, primary peritoneal epithelial cell tumor, cervical cancer, endometrial cancer, vaginal cancer, vulvar cancer, uterine cancer, and follicular solid cancer), malignant tumors of male reproductive tract (including testicular and penile cancer), kidney cancer (including renal cell carcinoma), brain cancer (including endogenous brain tumor, neuroblastoma, astrocytic brain tumor, glioma, metastatic tumor cell infiltration in the central nervous system), bone cancer (including osteoma and osteosarcoma), skin cancer (including melanoma, progressive tumors of human skin keratinocytes, squamous cell carcinoma), thyroid cancer, retinoblastoma, neuroblastoma, peritoneal leakage, malignant pleural leakage, mesothelioma, Wilms tumor, gallbladder cancer, trophoblastoma, hemangiopericytoma, and Kaposi's sarcoma. In some embodiments, the regimen of the present disclosure is suitable for treating non-small cell lung cancer or melanoma.

The "treatment" or "therapeutic effect" described in the present disclosure includes, for example, inhibiting or reducing the growth, infiltration or metastasis of tumor or cancer cells, etc., or reducing symptoms, prolonging the survival period, etc.

The subjects described herein may include, but are not limited to, vertebrates, mammals, rodents (e.g. hamsters, rats, mice), canines (e.g. dogs), felines (e.g. cats), horses, pigs, cattle, sheep, goats, primates (e.g. chimpanzees, apes or monkeys), or humans. The cells described herein may include, but are not limited to, cells of vertebrates, mammals, rodents (e.g. hamsters, rats, mice), canines (e.g. dogs), felines (e.g. cats), horses, pigs, cattle, sheep, goats, primates (e.g. chimpanzees, apes or monkeys), or humans.

In the present disclosure, the administration of endostatin and a treatment regimen for inducing DNA double-strand break can be performed simultaneously or sequentially. When performed sequentially, endostatin can be administered first, followed by a treatment regimen for inducing DNA double-strand break, or a treatment regimen for inducing DNA double-strand break can be performed first, followed by administration of endostatin.

The present disclosure also describes but not claim a method of inhibiting DNA-PKcs activity in a biological sample, comprising contacting the biological sample with endostatin. The "biological sample" described herein refers to a sample *in vitro* of a living organism, including but not limited to a cell culture or an extract thereof; a biopsy material obtained from a mammal or an extract thereof; and blood, saliva, urine, feces, semen, tears, or other body fluids or their extracts. Inhibition of DNA-PKcs activity in a biological sample can be used for a variety of purposes known to those skilled in the art. Examples include, but are not limited to, inhibition of DNA-PKcs in a bioassay. In one embodiment, the method of inhibiting DNA-PKcs activity in a biological sample is limited to a non-therapeutic method.

The term "pharmaceutically acceptable carrier" as used herein refers to substances that can be safely administered, such as solid or liquid diluents, fillers, antioxidants, and stabilizers. Depending on the route of administration, a variety of different carriers well known in the art can be administered, including, but not limited to, sugars, starches, celluloses and derivatives thereof, maltose, gelatin, talc, calcium sulfate, vegetable oils, synthetic oils, polyols, alginic acid, phosphate buffers, emulsifiers, isotonic saline, and/or pyrogen-free water.

As used herein, the term "combination of drugs" or "combination" includes various specific combinations of various drugs or treatment regimens that are used in combination. For example, when different drugs are used in combination, the various drugs used in combination may exist in the form of a mixture or a complex, or may exist in separate entities, respectively, and be administered to a subject simultaneously or sequentially. When a drug and a non-drug treatment regimen are used in combination, the drug and the treatment regimen can be administered to the subject simultaneously or sequentially.

The pharmaceutical composition, endostatin, and/or chemotherapeutic agent for inducing DNA double-strand break described herein can be administered in a therapeutically effective amount. The term "therapeutically effective amount" as used herein refers to an amount of an active compound sufficient to cause a biological or medical response desired by a clinician in a subject. In the present disclosure, the therapeutically effective amount of endostatin may be, for example, an amount capable of effectively inhibiting DNA-PKcs in tumor cells. In the present disclosure, the "therapeutically effective amount" of the pharmaceutical composition, endostatin, and/or chemotherapeutic agent for inducing DNA double-strand break can be determined by those skilled in the art according to factors such as the route of administration, the body weight, age, and condition of a subject. For example, for a pharmaceutical composition, endostatin and/or a chemotherapeutic agent, a typical daily dose may range from 0.01 mg to 100 mg of an active ingredient per kg of body weight, and for radiotherapy, a typical daily dose can range from 1 to 5 Gy.

The drug provided by the present disclosure can be formulated into a clinically acceptable dosage form such as powder and injection. The subject may be administered with the pharmaceutical composition, endostatin, and/or chemotherapeutic agent of the present disclosure by any suitable route, for example, oral administration, intravenous infusion, intramuscular injection, subcutaneous injection, subperitoneal administration, rectal administration, sublingual administration, or inhalation, transdermal administration.

The various methods mentioned in the present disclosure can be performed *in vivo* or *in vitro.*

The various methods mentioned in the present disclosure may be non-therapeutic.

Unless otherwise stated, scientific and technical terms used in this specification shall have the meanings commonly understood by those of ordinary skill in the art. In general, nomenclatures and techniques associated with cell and tissue culture, molecular biology, immunology, microbiology, genetics, and protein and nucleic acid chemistry as used in this specification are well known and commonly used in the art.

Unless otherwise stated, the methods and techniques used in this specification are generally performed according to the well known and routine methods in the art and in the manner described in the various references set forth or cited in this specification.

### Examples

In the following examples, the endostatin used is a recombinant natural human endostatin expressed by *E. coli,* which is from Beijing Protgen Biotechnology Development Co., Ltd. (Protgen) and has the following amino acid sequence:
MHSHRDFQPVLHLVALNSPLSGGMRGIRGADFQCFQQARAVGLAGTFRAFLSSR LQDLYSIVRRADRAAVPIVNLKDELLFPSWEALFSGSEGPLKPGARIFSFDGKDVLRH PTWPQKSVWHGSDPNGRRLTESYCETWRTEAPSATGQASSLLGGRLLGQSAASCHH AYIVLCIENSFMTASK (SEQ ID NO: 2); wherein the Met at the N-terminus is sometimes partially deleted when expressed by *E. coli.*

The ZBP-Endostatin used is an endostatin variant obtained by adding 9 additional amino acids (MGGSHHHHH) to the N-terminus of the natural human endostatin (Fu Y et al. Biochemistry 2010, 49, 6420-6429), provided by Beijing Protgen Biotechnology Development Co., Ltd. (Protgen), and the amino acid sequence is as follows:
MGGSHHHHHHSHRDFQPVLHLVALNSPLSGGMRGIRGADFQCFQQARAVGLA GTFRAFLSSRLQDLYSIVRRADRAAVPIVNLKDELLFPSWEALFSGSEGPLKPGARIFS FDGKDVLRHPTWPQKSVWHGSDPNGRRLTESYCETWRTEAPSATGQASSLLGGRLL GQSAASCHHAYIVLCIENSFMTASK (SEQ ID NO: 3); wherein the Met at the N-terminus is sometimes partially deleted when expressed by *E. coli.*

The PEG-Endostatin used is a polyethylene glycol (PEG) modified natural human endostatin, which is a product obtained by modifyingthe natural human endostatin molecule with a monomethoxypolyethylene glycol propionaldehyde (mPEG-ALD) with a molecular weight of 20 kDa, wherein the coupling site is the activated mPEG-ALD aldehyde group and N-terminal α-amino group of Endostatin. It was provided by Beijing Protgen Biotechnology Development Co., Ltd. (Protgen).

Non-small cell lung cancer H1299 (P53 deficient), A549 (P53 wild type) cell line, and melanoma MDA-MB-435S (P53 mutant) and A375 (P53 wild type) cell line were purchased from the National Experimental Cell Resource Sharing Platform (China Infrastructure of Cell Line Resource). The P53 stably overexpressing H1299 cell line (H1299-P53) and the P53 stably knockdown A549 cell line (A549-NC) were constructed using a lentivirus kit (purchased from Shanghai Genepharma), wherein the P53 sequence targeted by the shRNA used to knock down P53 in A549 cells was 5'-GACTCCAGTGGTAATCTAC-3' (SEQ ID NO: 4). Virus transfection and the construction of stable cell lines were performed according to the kit instructions. H1299-NC and A549-NC were stable cell lines obtained by transfecting H1299 cells and A549 cells with blank lentivirus, respectively.

### Example 1. Endostatin interacted directly with DNA-PKcs.

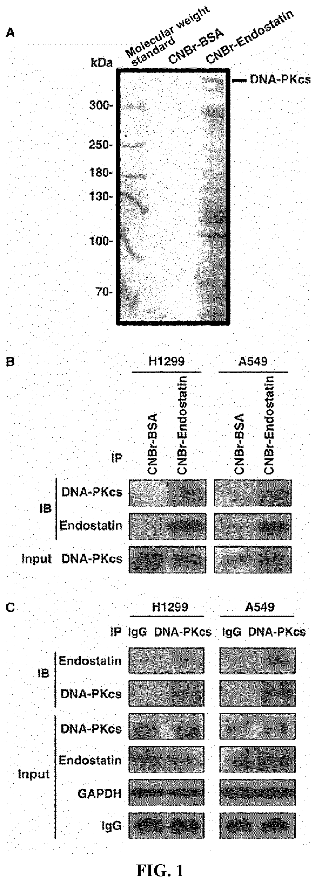

CNBr-activated Sepharose 4B used in this example was purchased from Sigma. First, according to the instructions, endostatin and bovine serum albumin (BSA) were coupled to CNBr-activated Sepharose 4B, respectively, and incubated with A549 cell lysate. The pull down proteins were subjected to SDS-PAGE electrophoresis and stained with Coomassie blue. By Mass spectrometric analysis, DNA-PKcs was identified as a protein interacted with Endostatin (Figure 1A). Then, immunoblotting experiments demonstrated that the DNA-PKcs protein in both H1299 and A549 cell lysates can be precipitated by CNBr-activated Sepharose 4B coupled with Endostatin (Figure 1B). To further verify the interaction between the two, H1299 and A549 cells were incubated with Endostatin for 2 hours for endocytosis. The cell lysate was mixed with the antibody of DNA-PKcs, and then co-incubated with protein A/G beads. The immunoblotting assay verified that Endostatin could be co-precipitated by DNA-PKcs, which proved the interaction between the two (Figure 1C) .

### Example 2. Endostatin inhibited the activation of DNA-PKcs signaling pathway in tumor cells induced by a chemotherapeutic agent.

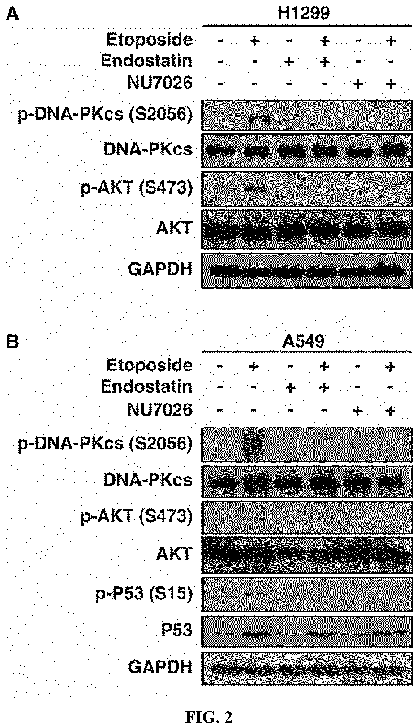

In this example, the known substrates of DNA-PKcs, AKT and P53, were used as detection indicators to verify the inhibitory effect of Endostatin on the DNA-PKcs pathway. Tumor cells (including non-small cell lung cancer H1299 and A549 cells) were divided into six treatment groups. The first group: negative control; the second group: Etoposide (10 µM, 1 h) treatment; the third group: Endostatin (10 µg/ml, 1 h) treatment; the fourth group: combined treatment with Endostatin (10 µg/ml, added 1 h before Etoposide) and Etoposide (10 µM, 1 h); the fifth group: NU7026 (10 µM, 1 h) treatment; the sixth group: combined treatment with NU7026 (10 µM, added 1 h before Etoposide) and Etoposide ( 10 µM, 1 h), wherein, NU7026 was a DNA-PKcs inhibitor. The immunoblotting assay results showed (Figure 2, wherein p-DNA-PKcs (S2056) represents the phosphorylation level of DNA-PKcs, S2056 represents its phosphorylation site; wherein p-AKT (S473) represents the phosphorylation level of AKT, and S473 represents its phosphorylation site; wherein p-P53 (S15) represents the phosphorylation level of P53, and S15 represents its phosphorylation site), compared with the control group, Etoposide treatment activated the level of the DNA-PKcs signaling pathway; Endostatin treatment did not affect the level of the DNA-PKcs signaling pathway; the combination of Endostatin and Etoposide inhibited activation of the DNA-PKcs signaling pathway by Etoposide. NU7026 treatment did not affect the level of the DNA-PKcs signaling pathway; the combination of NU7026 and Etoposide inhibited activation of the DNA-PKcs signaling pathway by Etoposide.

### Example 3. Endostatin delayed DNA-PKcs-mediated repair of DNA damage.

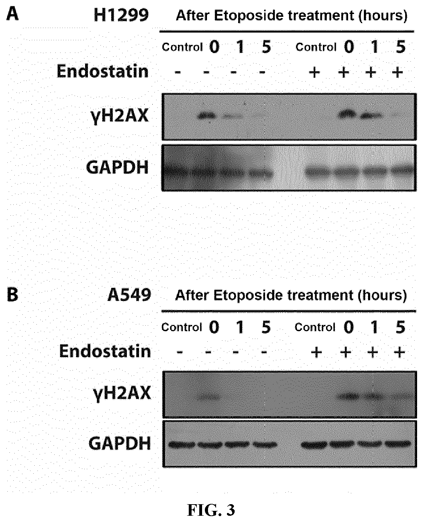

In this example, the level of γH2AX was used to reflect the degree of DSBs damage. Tumor cells (including H1299 and A549 cells) were divided into eight treatment groups. The first group: negative control, cells were collected at 0 hour; the second group: cells were collected after Etoposide (10 µM) treatment for 1 hour; the third group: after Etoposide (10 µM) treatment for 1 hour, it was replaced with fresh medium, and cells were collected after 2 hours; the fourth group, aftet Etoposide (10 µM) treatment for 1 hour, it was replaced with fresh medium, and cells were collected after 5 h; the fifth to eighth groups were first pretreated with Endostatin (10 µg / ml) for 1 h, and the subsequent treatments corresponded to those of the first to fourth groups, respectively. The immunoblotting assay results showed that the DNA damage produced by Etoposide-treated cells was gradually repaired after the drug was removed from the medium in the first to fourth groups, and the γH2AX level returned to baseline after 5 hours. In the fifth to eighth group, the use of Endostatin in combination delayed the reduction of γH2AX level, which proved that the repair of DSBs damage in cells was delayed (Figure 3).

### Example 4. Endostatin regulated the sensitivity of tumor cells to chemotherapy depending on the presence of DNA-PKcs

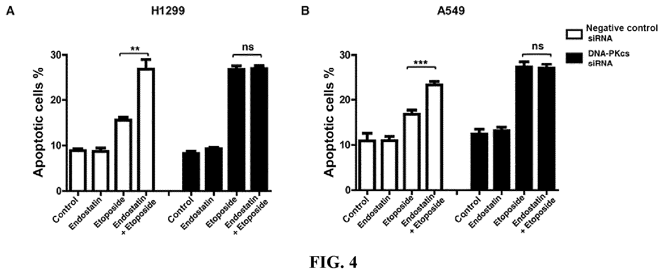

In this example, tumor cells (including H1299 and A549 cells) were divided into eight treatment groups. In the first four groups, cells were transfected with control siRNA, and in the last four groups, cells were transfected with siRNA that specifically knocked down DNA-PKcs. The siRNA sequence of DNA-PKcs is: forward 5'-CAGGGUUUAAUCAGAAUAUTT-3' (SEQ ID NO: 5), reverse 5'-AUAUUCUGAUUAAACCCUGTT-3' (SEQ ID NO: 6). The siRNA transfection method was performed according to the instructions of the transfection reagent Lipofectamine 2000. Then, in the eight groups, the cells were treated with drugs. Groups 1 and 4: control group; Groups 2 and 5: Endostatin (10 µg/ml, 16 h) treatment; Groups 3 and 6: Etoposide (10 µM for H1299, 1 µM for A549, 16 h) treatment; Groups 4 and 8: combined treatment with Endostatin (10 µg/ml, added 1 h before Etoposide) and Etoposide (10 µM for H1299, 1 µM for A549, 16 h). Cells were collected and operations were performed according to the instructions of Annexin V-FITC/PI Apoptosis Detection Kit. Apoptosis was detected by flow cytometry. A549 cells were P53 wild-type cells, and H1299 cells were P53-deficient cells. The results showed that A549 cells were more sensitive to the chemotherapeutic agent, and when the same degree of apoptosis was caused, the Etoposide concentration in H1299 cells was much higher than that in A549 cells. On the basis of this, the addition Endostatin would further promote Etoposide-induced apoptosis (Figure 4). After knocking down DNA-PKcs, the role of Endostatin in promoting apoptosis induced by Etoposide was weakened.

### Example 5. Endostatin synergized with Etoposide inhibited the activity of P53-deficient tumor cells.

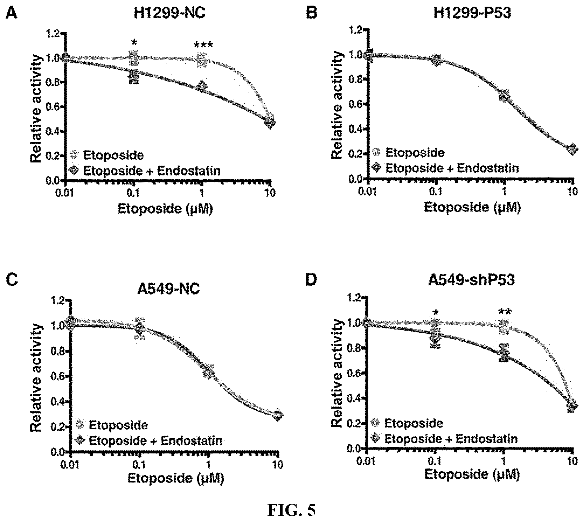

First, a lentivirus kit (purchased from Shanghai Genepharma) was used to construct a P53 stably overexpressing H1299 cell line (H1299-P53) and a P53 stably knockdown A549 cell line (A549-NC). The P53 sequence targeted by the shRNA used to knock down P53 in A549 cells was 5'-GACTCCAGTGGTAATCTAC-3' (SEQ ID NO: 4). Virus transfection and the construction of stable cell lines were performed according to the kit instructions. The constructed tumor cells (H1299-NC, H1299-P53, A549-NC, A549-shP53) were seeded into 96-well plates (1000 cells per well) and divided into eight treatment groups. In the first group to the fourth group, a medium containing different concentrations of Etoposide (0 µM, 0.1 µM, 1 µM, 10 µM) was added to each well. The fifth to eighth groups were first pretreated with a medium containing 10 µg/ml Endostatin for 1 h, and then a medium containing different Etoposide concentrations was added so that the final concentrations of Etoposide in the fifth to eighth groups were 0 µM, 0.1 µM, 1 µM, and 10 µM, respectively. After treatment for 12 hours, the drug-containing medium was replaced with a normal medium and the cells were further cultured for 72 hours. Cell activity was measured according to the instructions of the CCK8 kit (Dojindo, Tokyo, Japan). The results showed that for P53-deficient tumor cells (Figures 5A and D), Endostatin could enhance the toxicity of Etoposide to tumor cells. For tumor cells with normal P53 function (Figure 5B and C), Etoposide could significantly inhibit the activity of tumor cells, and Endostatin did not significantly enhance the inhibitory effect of Etoposide.

### Example 6. Endostatin synergized with Etoposide inhibited the survival of P53-deficient tumor cells.

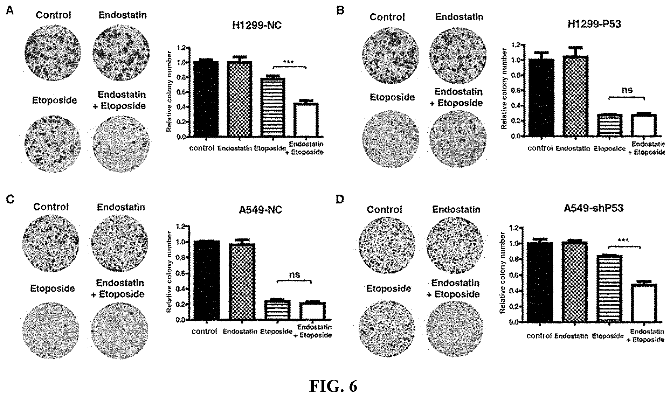

Tumor cells (H1299-NC, H1299-P53, A549-NC, A549-shP53) were divided into four treatment groups. The first group: negative control; the second group: Endostatin (10 µg/ml, 16 h) treatment; the third group: Etoposide (1 µM, 16 h) treatment; the fourth group: combined treatment with Endostatin (10 µg/ml, added 1 h before Etoposide) and Etoposide (1 µM, 16 h). After the treatment, the cells were digested with trypsin, counted, and re-plated in a six-well plate with 1000 cells per well. The cells were cultured in a normal medium for 14 days to form cell colonies. The results of cell colony counting showed that for P53-deficient tumor cells (Figures 6A and D), Endostatin could enhance the inhibitory effect of Etoposide on tumor cell colony-forming ability and reduce the viability of tumor cells. For tumor cells with normal P53 function (Figure 6B and C), Etoposide could significantly inhibit the colony-forming ability of tumor cells, and Endostatin did not significantly enhance theinhibitory effect of Etoposide.

### Example 7. Endostatin synergized with Etoposide promoted the apoptosis of P53-deficient tumor cells.

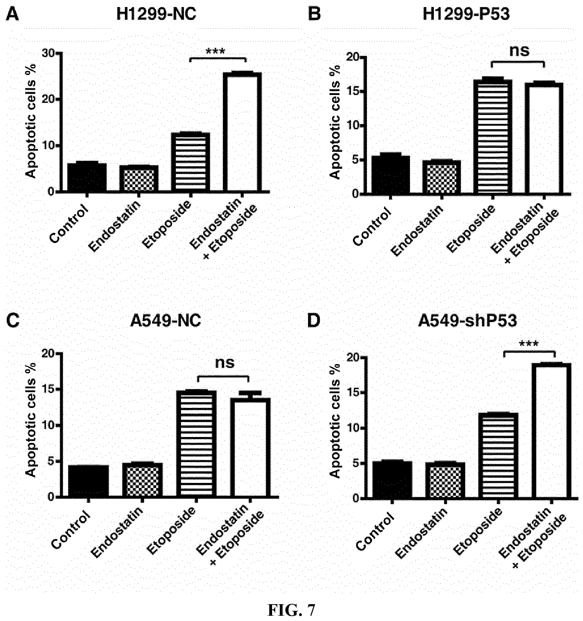

Tumor cells (H1299-NC, H1299-P53, A549-NC, A549-shP53) were divided into four treatment groups. The first group: negative control; the second group: Endostatin (10 µg/ml, 16 h) treatment; the third group: Etoposide (1 µM, 16 h) treatment; the fourth group: combined treatment with Endostatin (10 µg/ml, added 1 h before Etoposide) and Etoposide (1 µM, 16 h). The apoptotic cells and adherent cells floating in the culture medium were collected, and the cells were suspended in 500 µl PBS containing 0.05 mg/ml propidium iodide (PI), stained for 15 min at room temperature in dark, and detected by flow cytometry (BD FACS Calibur). PI-positive cells represent apoptotic cells. The results showed that for P53-deficient tumor cells (Figure 7A and D), Endostatin could enhance the ability of Etoposide to promote the apoptosis of tumor cells. For tumor cells with normal P53 function (Figure 7B and C), Etoposide could significantly promote the apoptosis of tumor cells, and Endostatin did not significantly enhance the pro-apoptotic effect of Etoposide.

### Example 8. Endostatin synergized Doxorubicin inhibited the activity of P53-deficient tumor cells.

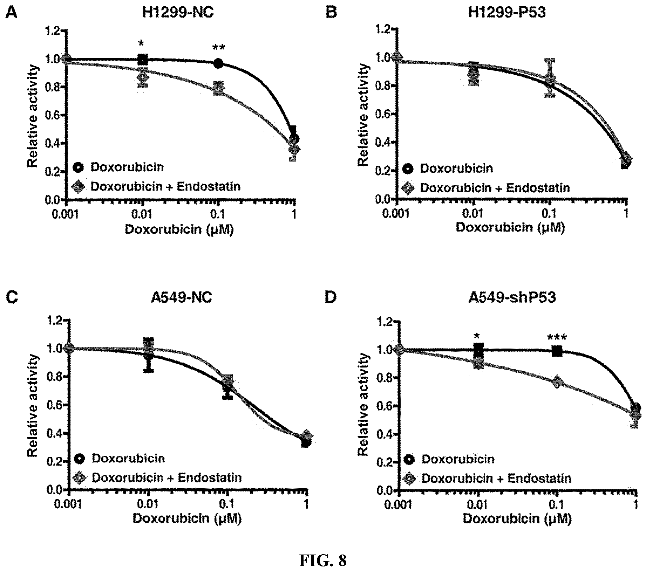

In this example, another chemical drug, Doxorubicin, was used to verify that Endostatin can enhance the sensitivity of P53-deficient tumor cells to chemotherapy. Operation process was the same as in Example 5. The results showed that for P53-deficient tumor cells (H1299-NC and A549-shP53) (Figures 8A and D), Endostatin could enhance the toxicity of Doxorubicin to tumor cells. For tumor cells with normal P53 function (H1299-P53 and A549-NC) (Figure 8B and C), Doxorubicin could significantly inhibit the activity of tumor cells, and Endostatin did not significantly enhance the inhibitory effect of Doxorubicin.

### Example 9. The inhibitory effect of Etoposide synergized with Endostatin was applicable to other types of tumor cells.

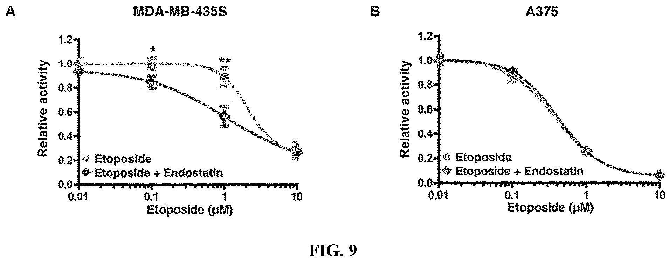

This example used two cell lines of another tumor type (melanoma), the MDA-MB-435S (P53 mutant) and A375 (P53 wild type) cell lines, to verify the regulatory function of Endostatin on the chemotherapeutic sensitivity of other types of tumor cells with different P53 status. Operation process was the same as in Example 5. The results showed that for MDA-MB-435S cells (Figures 9A and D), Endostatin could enhance the toxicity of Etoposide to tumor cells. For A375 cells (Figure 9B and C), Etoposide could significantly inhibit the activity of tumor cells, and Endostatin did not significantly enhance the inhibitory effect of Etoposide.

### Example 10. Endostatin inhibited DNA-PKcs to repair cell damage, induced activation of apoptotic pathway and degradation of DNA-PKcs in P53-deficient tumor cells.

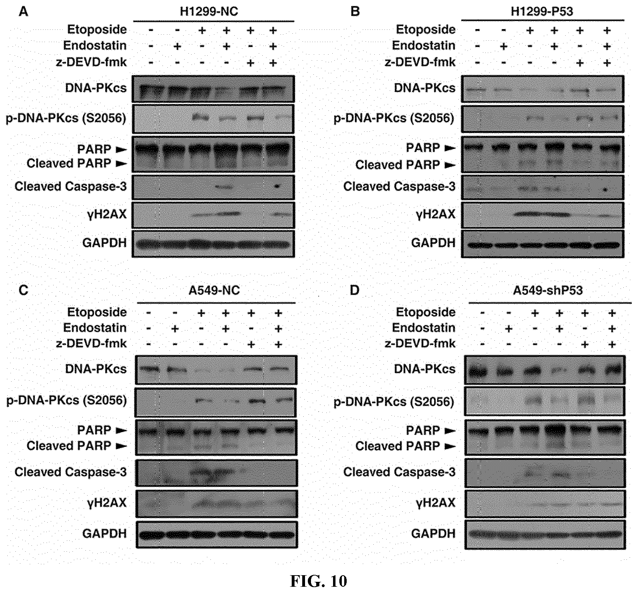

Studies have shown that DNA-PKcs is a substrate of Caspase-3, and activated Caspase-3 will cleave DNA-PKcs to degrade it (T. Itoh et al. Journal of dermatological science, 2001; 25, 72-77, K. M. Henkels et al. Cancer research, 1997; 57, 4488-4492). In this example, tumor cells (H1299-NC, H1299-P53, A549-NC, A549-shP53) were divided into six treatment groups. The first group: negative control; the second group: Endostatin (10 µg/ml, 16 h) treatment; the third group: Etoposide (10 µM, 16 h) treatment; the fourth group: combined treatment with Endostatin (10 µg/ml, added 1 h before Etoposide) and Etoposide (10 µM, 16 h); the fifth group: combined treamtment with z-DEVD-fmk (10 µM, added 1 h before Etoposide) and Etoposide (10 µM, 16 h); the sixth group: combined treatment with z-DEVD-fmk (10 µM) and Endostatin (10 µg/ml) added 1 h before Etoposide, and then Etoposide (10 µM, 16 h), wherein, z-DEVD-fmk was a caspase-3 inhibitor. Immunoblotting assay detected DNA-PKcs, PARP, γH2AX and Caspase-3 signaling pathways. The results (as shown in Figure 10, in which p-DNA-PKcs (S2056) represents the phosphorylation level of DNA-PKcs, and S2056 represents its phosphorylation site) showed that compared with the control group, in both P53-deficient tumor cells (Figures 10A and D) and tumor cells with normal P53 function (Figures 10B and C), Endostatin did not affect the above-mentioned signaling pathway levels; for P53-deficient tumor cells, Etoposide had no significant effect on the above signaling pathways; as compared with Etoposide, the combination of Endostatin and Etoposide significantly promoted the activation of Caspase-3, and degradation of DNA-PKcs and PARP, with a significant increase in γH2AX; the combination of z-DEVD-fmk and Etoposide had no significant effect on the above signal levels; z-DEVD-fmk could reverse the synergistic effect of Endostatin and Etoposide to some extent. For tumor cells with normal P53 function, both the treatment with Etoposide alone and the combined treatment with Endostatin and Etoposide showed significant activation of Caspase-3, degradation of DNA-PKcs and PARP, and an increase in γH2AX; the combination of z-DEVD-fmk and Etoposide reversed the effect of Etoposide to some extent; the combination of z-DEVD-fmk, Endostatin and Etoposide showed that z-DEVD-fmk reversed the effect of Etoposide, the protein level of DNA-PKcs was restored, and the synergistic effect of Endostatin and Etoposide was reflected to a certain extent.

### Example 11. Endostatin synergized with Etoposide up-regulated the expression of pro-apoptotic molecules in P53-deficient tumor cells.

Tumor cells (H1299-NC, H1299-P53, A549-NC, A549-shP53) were divided into four treatment groups. The first group: negative control; the second group: Endostatin (10 µg/ml, 16 h) treatment; the third group: Etoposide (1 µM, 16 h) treatment; the fourth group: combined treatment with Endostatin (10 µg/ml, added 1 h before Etoposide) and Etoposide (1 µM, 16 h). After the cells were treated as described above, the total RNA of the cells was extracted, and the relative changes in mRNA levels of BAX, NOXA, PUMA, and P21 in the cells were detected by real-time PCR. The primers used are shown in the following table.

| Gene | Forward primer (5'-3') | Reverse primer (5'-3') |
|---|---|---|
| *BAX* | atggacgggtccggggagcagccc (SEQ ID NO: 7) | ggtgagcactcccgccacaaagat (SEQ ID NO: 8) |
| *NOXA* | actgttcgtgttcagctc (SEQ ID NO: 9) | gtagcacactcgacttcc (SEQ ID NO: 10) |
| *PUMA* | gacctcaacecacagta (SEQ | ctaattgggctccatct (SEQ ID |

| | | |
|---|---|---|
| | ID NO: 11) | NO: 12) |
| *P21* | caccgagacaccactggagg (SEQ ID NO: 13) | gagaagatcagccggcgttt (SEQ ID NO: 14) |
| *GAPDH* | caaggtcatccatgacaactttg (SEQ ID NO: 15) | gtccaccaccctgttgctgtag (SEQ ID NO: 16) |

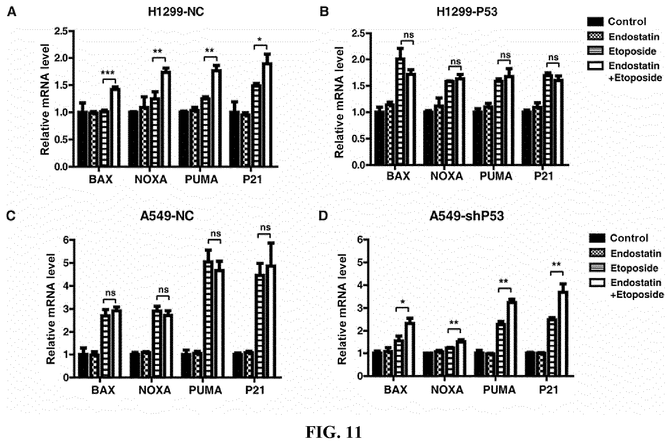

The results showed that for P53-deficient tumor cells (Figure 11A and D), Endostatin could enhance Etoposide to up-regulate pro-apoptotic molecules. For tumor cells with normal P53 function (Figures 11B and C), Etoposide could significantly up-regulate the above pro-apoptotic molecules, and Endostatin did not significantly enhance the pro-apoptotic effect of Etoposide.

### Example 12. Endostatin enhanced the inhibitory effect of Etoposide on the P53-deficient tumor growth.

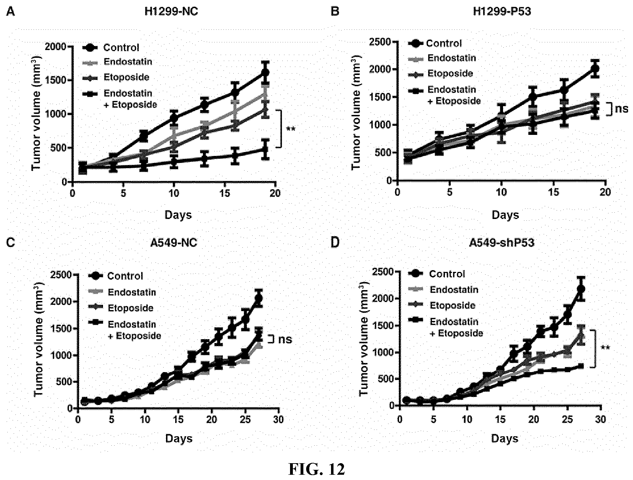

Tumor cells (H1299-NC, H1299-P53, A549-NC, A549-shP53) were inoculated subcutaneously into 6 to 8 week-old nude mice (Beijing Weitong Lihua Experimental Animal Technology Co., Ltd.). Tumor-bearing nude mice were randomly divided into four groups with 6 to 8 animals for each group, and different drug treatments were performed. The first group: negative control; the second group: Endostatin (20 mg/kg, intraperitoneal injection, once a day) treatment; the third group: Etoposide (2 mg/kg, intraperitoneal injection, once every two days) treatment; the fourth group: combined treatment with Endostatin (20 mg/kg, intraperitoneal injection, once a day) and Etoposide (2 mg/kg, intraperitoneal injection, once every two days). Tumor volume was measured every other day. 2 to 4 weeks after administration, the mice were sacrificed and the tumor tissue was removed for further use. The results showed that in the P53-deficient mouse tumor model (Figures 12A and D), as compared with the control group, the tumor suppression rates of Endostatin were 19.6% (H1299-NC, Day 19) and 37.1% (A549-shP53, Day 27); the tumor suppression rates of Etoposide were 28.0% (H1299-NC, Day 19) and 39.3% (A549-shP53, Day 27); the combination of Endostatin and Etoposide had a synergistic effect on inhibiting tumor growth, with tumor suppression rates of 66.9% (H1299-NC, Day 19) and 65.8% (A549-shP53, Day 27). In a mouse tumor model with normal P53 function (Figures 12B and C), as compared with the control group, the tumor suppression rates of Endostatin were 33.8% (H1299-P53, Day 19) and 40.9% (A549-NC, Day 27); the tumor suppression rates of Etoposide were 30.0% (H1299-P53, Day 19) and 34.0% (A549-NC, Day 27); Endostatin could not enhance the inhibitory effect of Etoposide on tumor growth, and the tumor suppression rates of the combination were 37.7% (H1299-P53, Day 19) and 32.2% (A549-NC, Day 27).

### Example 13. The combined use of Endostatin and Etoposide had a synergistic effect on prolonging the survival of P53-deficient tumor-bearing mice.

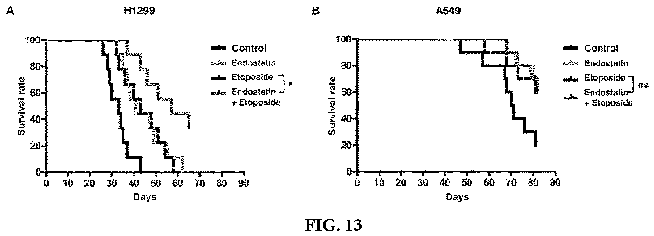

H1299 and A549 cells were cultured in a vigorous and proliferative state, and tumor collection, grouping, and administration methods were the same as in Example 12. The administration was stopped after 25 days, and the survival of the mice was recorded daily, and the survival curve of the mice was plotted. The results showed that in the H1299 mouse tumor model (Figure 13A), as compared with the control group, the median survival time of the Endostatin-treated group was 24.2% longer than that of the control group; the median survival time of the Etoposide treatment group was 30.3% longer than that of the control group; the combination of Endostatin and Etoposide had a synergistic effect on prolonging the survival time of mice, and the median survival time was 72.7% longer than that of the control group. In a mouse tumor model with normal P53 function (Figure 13B), as compared with the control group, the Endostatin treatment group and the Etoposide treatment group could prolong the survival time of the mice to some extent, and the combination of Endostatin and Etoposide had no synergistic effect on prolonging the survival time of mice.

### Example 14. Endostatin synergized with Etoposide inhibited the proliferation of P53-deficient tumors and the expression level of DNA-PKcs.

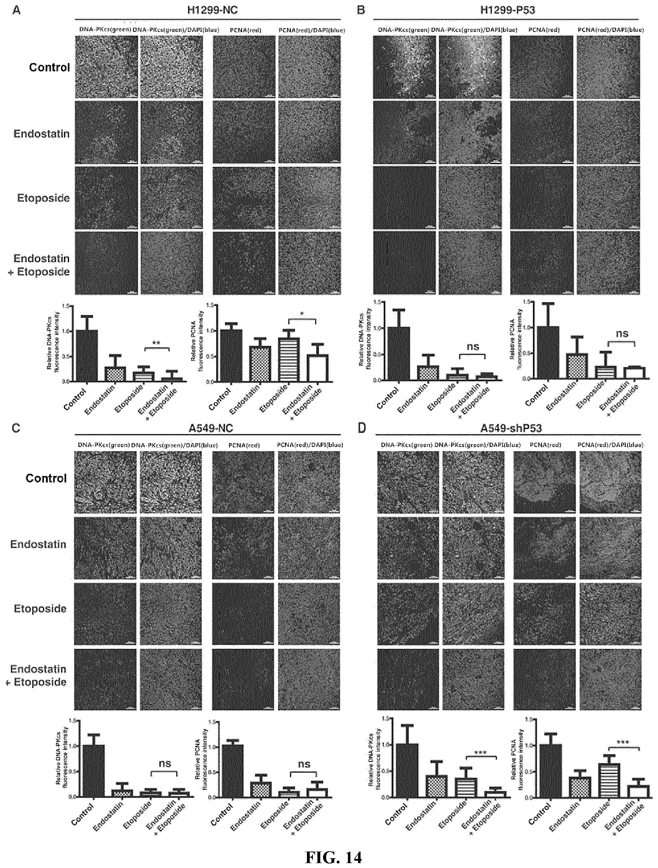

The tumor tissue removed in Example 12 was fixed and embedded, and then sectioned. PCNA (a marker molecule indicating the vigorous proliferation status of tumor cells) and DNA-PKcs protein in tumor tissues were stained by immunofluorescence technique, and were observed and imaged with a laser confocal microscope (Nikon A1). The experimental results are shown in Figure 14, in which blue represents the nucleus of DAPI-stained cells, green represents the DNA-PKcs protein, and red represents the PCNA protein. Taking the H1299-NC tumor tissue shown in Figure 14A as an example, the first column on the left represents DNA-PKcs single staining, and the green area represents the DNA-PKcs protein expression level. The second column on the left represents the results of DNA-PKcs (green) merged with nucleus (blue), and the overlapping part is shown in cyan. The third column on the left represents PCNA single staining, and the red area represents the PCNA protein expression level, indicating the strength of tumor cell proliferation. The fourth column on the left represents the result of the PCNA (red) merged with nucleus (blue), and the overlapping part is shown in purple. Similarly, Figures 14B, C, and D show the expression of PCNA and DNA-PKcs in H1299-P53, A549-NC, and A549-shP53 tumor tissues, respectively. The order of protein staining and image arrangement in the figure is consistent with Figure 12A. Quantitative results showed that in the P53-deficient mouse tumor model (Figures 14A and D), as compared with the control group, both Endostatin and Etoposide can attenuate tumor cell proliferation to a certain extent and reduce the protein level of DNA-PKcs; the combination of Endostatin and Etoposide can function synergistically, and can more significantly inhibit tumor proliferation and DNA-PKcs protein levels as compared with the Etoposide treatment group. In a mouse tumor model with normal P53 function (Figure 14B and C), as compared with the control group, Endostatin, Etoposide, and the combination of Endostatin and Etoposide could reduce the proliferation of tumor cells and reduce the protein level of DNA-PKcs, and Endostatin did not enhance the inhibitory effect of Etoposide on tumor proliferation and the effect of Etoposide on the protein levels of DNA-PKcs.

### Example 15. ZBP-Endostatin and PEG-Endostatin inhibited the activation of DNA-PKcs signaling pathway in tumor cells induced by a chemotherapeutical agent

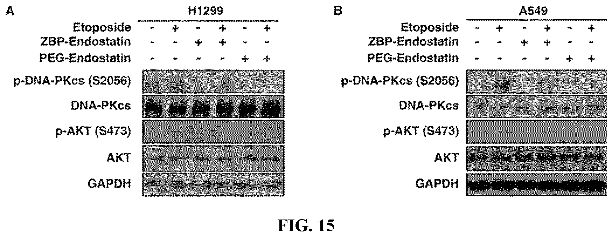

In this example, the inhibition of the DNA-PKcs pathway by ZBP-Endostatin and PEG-Endostatin was verified by detecting the activation levels of DNA-PKcs and AKT. Tumor cells (including H1299 and A549 cells) were divided into six treatment groups. The first group: negative control; the second group: Etoposide (10 µM, 1 h) treatment; the third group: ZBP-Endostatin (10 µg/ml, 1 h) treatment; the fourth group: combined treatment with ZBP-Endostatin (10 µg/ml, added 1 h before Etoposide) and Etoposide (10 µM, 1 h); the fifth group: PEG-Endostatin (10 µg/ml, 1 h) treatment; the sixth group: combined treatment with PEG-Endostatin (10 µg/ml, added 1 h before Etoposide) and Etoposide (10 µM, 1 h). Immunoblotting assay results showed (Figure 15) that as compared with the control group, Etoposide treatment activated the level of the DNA-PKcs signaling pathway in H1299 and A549 cells (as shown in the immunoblotting assay results of p-DNA-PKcs (S2056) and p-AKT (S473), wherein p-DNA-PKcs (S2056) represents the phosphorylation level of DNA-PKcs, and S2056 represents its phosphorylation site, wherein p-AKT represents the phosphorylation level of AKT, and S473 represents its phosphorylation site); the combination of ZBP-Endostatin or PEG-Endostatin and Etoposide inhibited the activation of the DNA-PKcs signaling pathway by Etoposide.

## Claims

1. A combination of drugs including an endostatin and a chemotherapeutic agent for use in inhibiting the activity of a tumor or cancer cell, wherein the tumor or cancer cell is deficient in P53 function, wherein the tumor or cancer cell is a melanoma cell and wherein the chemotherapeutic agent is able to induce DNA double strand break in the cell.

2. The combination of drugs for use according to claim 1, wherein the endostatin and the chemotherapeutic agent are administered simultaneously or sequentially.

3. The combination of drugs for use according to claim 1 or 2, wherein the chemotherapeutic agent is Etoposide or Doxorubicin.

4. The combination of drugs for use according to any one of claims 1 to 3, wherein the endostatin is:
a natural human endostatin;
an endostatin variant obtained by adding 9 additional amino acids MGGSHHHHH to the N-terminus of the natural human endostatin, wherein the Met at the N-terminus of the endothelin variant is sometimes partially deleted when expressed by E. coli; or
a product obtained by modifying a natural human endostatin with a monomethoxy polyethylene glycol propionaldehyde (mPEG-ALD) with a molecular weight of 20 kDa, wherein their coupling site is the activated mPEG-ALD aldehyde group and the N- terminal α-amino group of the natural human endostatin.

5. An endostatin for use in increasing sensitivity of a tumor or cancer cell to a treatment of a chemotherapeutic agent, wherein the cell is deficient in P53 function, and the chemotherapeutic agent is able to induce DNA double strand break in the cell, wherein the tumor or cancer cell is a non-small cell lung cancer cell or melanoma cell.

6. The endostatin for use according to claim 5, wherein the chemotherapeutic agent is Etoposide or Doxorubicin.

7. The endostatin for use according to claim 5 or 6, wherein the endostatin is contacted with the tumor or cancer cell simultaneously or sequentially with the chemotherapeutic agent, preferably, the endostatin is contacted with the tumor or cancer cell prior to or after the chemotherapeutic agent is contacted with the tumor or cancer cell.

8. The endostatin for use according to any one of claims 5-7, wherein the endostatin inhibits the repair of DNA double strand break induced by the chemotherapeutic agent in the tumor or cancer cell.

9. A combination for use in inducing apoptosis in a tumor or cancer cell, the combination comprising:
a) a chemotherapeutic agent; and
b) an endostatin,
wherein the tumor or cancer cell is deficient in P53 function, and the chemotherapeutic agent is able to induce DNA double strand break in the cell, wherein the tumor or cancer cell is a non-small cell lung cancer cell or melanoma cell.

10. The combination for use according to claim 9, wherein the chemotherapeutic agent is Etoposide or Doxorubicin.

11. The combination for use according to claim 9 or 10, wherein the endostatin is contacted with the tumor or cancer cell simultaneously or sequentially with the chemotherapeutic agent, preferably, the endostatin is contacted with the tumor or cancer cell prior to or after the chemotherapeutic agent is contacted with the tumor or cancer cell.

12. The combination for use according to any one of claims 9-11, wherein the endostatin inhibits the repair of DNA double strand break induced by the chemotherapeutic agent in the tumor or cancer cell.

13. The endostatin for use according to any one of claims 5-8 or the combination for use according to any one of claims 9-12, wherein the endostatin is:
a natural human endostatin;
an endostatin variant obtained by adding 9 additional amino acids MGGSHHHHH to the N-terminus of the natural human endostatin, wherein the Met at the N-terminus of the endothelin variant is sometimes partially deleted when expressed by E. coli; or
a product obtained by modifying a natural human endostatin with a monomethoxy polyethylene glycol propionaldehyde (mPEG-ALD) with a molecular weight of 20 kDa, wherein their coupling site is the activated mPEG-ALD aldehyde group and the N- terminal α-amino group of the natural human endostatin.

## Patentansprüche

1. Eine Kombination von Arzneimitteln, die ein Endostatin und ein chemotherapeutisches Mittel zur Verwendung beim Hemmen der Aktivität eines Tumors oder einer Krebszelle umfasst, wobei der Tumor oder die Krebszelle eine mangelhafte P53-Funktion aufweist, wobei der Tumor oder die Krebszelle eine Melanomzelle ist und wobei das chemotherapeutische Mittel in der Lage ist, einen DNA-Doppelstrangbruch in der Zelle zu induzieren.

2. Die Kombination von Arzneimitteln zur Verwendung gemäß Anspruch 1, wobei das Endostatin und das chemotherapeutische Mittel gleichzeitig oder nacheinander verabreicht werden.

3. Die Kombination von Arzneimitteln zur Verwendung gemäß Anspruch 1 oder 2, wobei das chemotherapeutische Mittel Etoposid oder Doxorubicin ist.

4. Die Kombination von Arzneimitteln zur Verwendung gemäß einem der Ansprüche 1 bis 3, wobei das Endostatin Folgendes ist:
ein natürliches menschliches Endostatin;
eine Endostatinvariante, die durch Hinzufügen von 9 zusätzlichen Aminosäuren MGGSHHHHH zum N-Terminus des natürlichen menschlichen Endostatins erhalten wird, wobei das Met am N-Terminus der Endothelinvariante manchmal teilweise deletiert wird, wenn es durch E. coli exprimiert wird; oder
ein Produkt, das durch Modifizieren eines natürlichen menschlichen Endostatins mit einem Monomethoxy-Polyethylenglycol-Propionaldehyd (mPEG-ALD) mit einem Molekulargewicht von 20 kDa erhalten wird, wobei ihre Kopplungsstelle die aktivierte mPEG-ALD-Aldehydgruppe und die N-terminale α-Aminogruppe des natürlichen menschlichen Endostatins ist.

5. Ein Endostatin zur Verwendung bei der Erhöhung der Empfindlichkeit eines Tumors oder einer Krebszelle gegenüber einer Behandlung mit einem chemotherapeutischen Mittel, wobei die Zelle eine mangelhafte P53-Funktion aufweist und wobei das chemotherapeutische Mittel in der Lage ist, einen DNA-Doppelstrangbruch in der Zelle zu induzieren, wobei der Tumor oder die Krebszelle eine nicht-kleinzellige Lungenkrebszelle oder Melanomzelle ist.

6. Das Endostatin zur Verwendung gemäß Anspruch 5, wobei das chemotherapeutische Mittel Etoposid oder Doxorubicin ist.

7. Das Endostatin zur Verwendung gemäß Anspruch 5 oder 6, wobei das Endostatin gleichzeitig oder nacheinander mit dem chemotherapeutischen Mittel mit der Tumor- oder Krebszelle in Kontakt gebracht wird, wobei das Endostatin vorzugsweise mit der Tumor- oder Krebszelle in Kontakt gebracht wird, bevor oder nachdem das chemotherapeutische Mittel mit der Tumor- oder Krebszelle in Kontakt gebracht wird.

8. Das Endostatin zur Verwendung gemäß einem der Ansprüche 5-7, wobei das Endostatin die Reparatur eines durch das chemotherapeutische Mittel in der Tumor- oder Krebszelle induzierten DNA-Doppelstrangbruchs hemmt.

9. Eine Kombination zur Verwendung bei der Induktion von Apoptose in einer Tumor- oder Krebszelle, wobei die Kombination Folgendes aufweist:
a) ein chemotherapeutisches Mittel; und
b) ein Endostatin,
wobei die Tumor- oder Krebszelle eine mangelhafte P53-Funktion aufweist und wobei das chemotherapeutische Mittel in der Lage ist, einen DNA-Doppelstrangbruch in der Zelle zu induzieren, wobei die Tumor- oder Krebszelle eine nicht-kleinzellige Lungenkrebszelle oder Melanomzelle ist.

10. Die Kombination zur Verwendung gemäß Anspruch 9, wobei das chemotherapeutische Mittel Etoposid oder Doxorubicin ist.

11. Die Kombination zur Verwendung gemäß Anspruch 9 oder 10, wobei das Endostatin gleichzeitig oder nacheinander mit dem chemotherapeutischen Mittel mit dem Tumor oder der Krebszelle in Kontakt gebracht wird, wobei das Endostatin vorzugsweise mit dem Tumor oder der Krebszelle in Kontakt gebracht wird, bevor oder nachdem das chemotherapeutische Mittel mit dem Tumor oder der Krebszelle in Kontakt gebracht wird.

12. Die Kombination zur Verwendung gemäß einem der Ansprüche 9-11, wobei das Endostatin die Reparatur des durch das chemotherapeutische Mittel in der Tumor- oder Krebszelle induzierten DNA-Doppelstrangbruchs hemmt.

13. Das Endostatin zur Verwendung gemäß einem der Ansprüche 5-8 oder die Kombination zur Verwendung gemäß einem der Ansprüche 9-12, wobei das Endostatin Folgendes ist:
ein natürliches menschliches Endostatin;
eine Endostatinvariante, die durch Hinzufügen von 9 zusätzlichen Aminosäuren MGGSHHHHH zum N-Terminus des natürlichen menschlichen Endostatins erhalten wird, wobei das Met am N-Terminus der Endothelinvariante manchmal teilweise deletiert wird, wenn es durch E. coli exprimiert wird; oder
ein Produkt, das durch Modifizierung eines natürlichen menschlichen Endostatins mit einem Monomethoxy-Polyethylenglycol-Propionaldehyd (mPEG-ALD) mit einem Molekulargewicht von 20 kDa erhalten wird, wobei ihre Kopplungsstelle die aktivierte mPEG-ALD-Aldehydgruppe und die N-terminale α-Aminogruppe des natürlichen menschlichen Endostatins ist.

## Revendications

1. Combinaison de médicaments comportant une endostatine et un agent chimiothérapeutique pour son utilisation dans l'inhibition de l'activité d'une tumeur ou cellule cancéreuse, dans laquelle la tumeur ou cellule cancéreuse est déficiente en fonction P53, dans laquelle la tumeur ou cellule cancéreuse est une cellule de mélanome et dans laquelle l'agent chimiothérapeutique est capable d'induire une cassure double brin d'ADN dans la cellule.

2. Combinaison de médicaments destinée à être utilisée selon la revendication 1, dans laquelle l'endostatine et l'agent chimiothérapeutique sont administrés simultanément ou séquentiellement.

3. Combinaison de médicaments destinée à être utilisée selon la revendication 1 ou 2, dans laquelle l'agent chimiothérapeutique est l'étoposide ou la doxorubicine.

4. Combinaison de médicaments destinée à être utilisée selon l'une quelconque des revendications 1 à 3, dans laquelle l'endostatine est :
une endostatine humaine naturelle ;
une variante d'endostatine obtenue par l'ajout de 9 acides aminés supplémentaires MGGSHHHHH au N-terminal de l'endostatine humaine naturelle, dans laquelle le Met au N-terminal de la variante d'endothéline est parfois partiellement supprimé lorsqu'il est exprimé par E. coli ; ou
un produit obtenu par modification d'une endostatine humaine naturelle avec un monométhoxy polyéthylène glycol propionaldéhyde (mPEG-ALD) avec un poids moléculaire de 20 kDa, dans laquelle leur site de couplage est le groupe aldéhyde mPEG-ALD activé et le groupe α-amino N-terminal de l'endostatine humaine naturelle.

5. Endostatine destinée à être utilisée dans l'augmentation de la sensibilité d'une tumeur ou cellule cancéreuse à un traitement d'un agent chimiothérapeutique, dans laquelle la cellule est déficiente en fonction P53, et l'agent chimiothérapeutique est capable d'induire une cassure double brin d'ADN dans la cellule, dans laquelle la tumeur ou cellule cancéreuse est une cellule de cancer du poumon non à petites cellules ou une cellule de mélanome.

6. Endostatine destinée à être utilisée selon la revendication 5, dans laquelle l'agent chimiothérapeutique est l'étoposide ou la doxorubicine.

7. Endostatine destinée à être utilisée selon la revendication 5 ou 6, dans laquelle l'endostatine est mise en contact avec la tumeur ou cellule cancéreuse simultanément ou séquentiellement avec l'agent chimiothérapeutique, de préférence, l'endostatine est mise en contact avec la tumeur ou cellule cancéreuse avant ou après que l'agent chimiothérapeutique soit mis en contact avec la tumeur ou cellule cancéreuse.

8. Endostatine destinée à être utilisée selon l'une quelconque des revendications 5-7, dans laquelle l'endostatine inhibe la réparation de la cassure double brin d'ADN induite par l'agent chimiothérapeutique dans la tumeur ou cellule cancéreuse.

9. Combinaison destinée à être utilisée pour induire l'apoptose dans une tumeur ou cellule cancéreuse, la combinaison comprenant :
a) un agent chimiothérapeutique ; et
b) une endostatine,
dans laquelle la tumeur ou cellule cancéreuse est déficiente en fonction P53, et l'agent chimiothérapeutique est capable d'induire une cassure double brin d'ADN dans la cellule, dans laquelle la tumeur ou cellule cancéreuse est une cellule de cancer du poumon non à petites cellules ou une cellule de mélanome.

10. Combinaison destinée à être utilisée selon la revendication 9, dans laquelle l'agent chimiothérapeutique est l'étoposide ou la doxorubicine.

11. Combinaison destinée à être utilisée selon la revendication 9 ou 10, dans laquelle l'endostatine est mise en contact avec la tumeur ou cellule cancéreuse simultanément ou séquentiellement avec l'agent chimiothérapeutique, de préférence, l'endostatine est mise en contact avec la tumeur ou cellule cancéreuse avant ou après que l'agent chimiothérapeutique soit mis en contact avec la tumeur ou cellule cancéreuse.

12. Combinaison destinée à être utilisée selon l'une quelconque des revendications 9-11, dans laquelle l'endostatine inhibe la réparation de la cassure double brin d'ADN induite par l'agent chimiothérapeutique dans la tumeur ou cellule cancéreuse.

13. Endostatine destinée à être utilisée selon l'une quelconque des revendications 5-8 ou combinaison destinée à être utilisée selon l'une quelconque des revendications 9-12, dans laquelle l'endostatine est :
une endostatine humaine naturelle ;
une variante d'endostatine obtenue par l'ajout de 9 acides aminés supplémentaires MGGSHHHHH au N-terminal de l'endostatine humaine naturelle, dans laquelle le Met au N-terminal de la variante d'endothéline est parfois partiellement supprimé lorsqu'il est exprimé par E. coli ; ou
un produit obtenu par modification d'une endostatine humaine naturelle avec un monométhoxy polyéthylène glycol propionaldéhyde (mPEG-ALD) avec un poids moléculaire de 20 kDa, dans laquelle leur site de couplage est le groupe aldéhyde mPEG-ALD activé et le groupe α-amino N-terminal de l'endostatine humaine naturelle.
